# EUROPEAN PATENT APPLICATION

(11) **EP 1 323 731 A1**
(43) Date of publication of application: **02.07.2003**
(21) Application number: 01970293.5
(22) Date of filing: 28.09.2001
(51) Int. Cl.: C07K 14/00, C12N 15/85, A61K 48/00

(54) **NOVEL PEPTIDES**

(30) Priority: 12.10.2000 JP 2000312600
(71) Applicant: MOCHIDA PHARMACEUTICAL CO., LTD., Shinjuku-ku Tokyo 160-8515 (JP)
(72) Inventor: KURIYAMA, Shinichi, c/o. MOCHIDA PHARM. CO., LTD, Shinjuku-ku, Tokyo 160-8515 (JP); TAGUCHI, Yasushi, c/o. MOCHIDA PHARM. CO., LTD., Shinjuku-ku, Tokyo 160-8515 (JP)
(74) Representative: Grünecker, Kinkeldey, Stockmair & Schwanhäusser Anwaltssozietät
(86) International application number: JP0108565
(87) International publication number: WO02030961

(57) **Abstract**

Peptides which are capable of binding to a gene and transferring this gene into cells, have an affinity with phosphatidylserine, have a high safety and an excellent selectivity, can be easily formulated into a complex with the gene and can serve as a vector having a high gene transfer efficiency into cells.

## Description

### Technical Field

This invention relates to a peptide including a sequence of 18 amino acids which is constituted from alternately arranged two hydrophobic sides and two hydrophilic sides in α-helix structural model depicted by Edmundson wheel plots, wherein at least one of the two hydrophilic sides is a positively charged side.

This invention also relates to a vector for gene therapy containing such peptide; a composition for gene therapy containing such peptide and a plasmid having herpes simplex virus thymidine kinase gene inserted therein; and a method for treating or preventing cancer, tumor, arterial sclerosis, restenosis after percutaneous transluminal coronary angioplasty, or GVHD.

This invention also relates to a method for delivering a substance which binds to such peptide into a cell, wherein such peptide is used as a carrier.

### Background Art

Entire genome sequences is being determined in an increasing number of pathogenic microorganisms, pathogenic viruses, and human, and various attempts are being made to treat various diseases by using the genetic information obtained. One such attempt is the treatment wherein a DNA, an RNA, or a derivative, a modification, or an analog thereof, namely, a nucleic acid is administered to the interior of the body. Through such administration of the nucleic acid to the body, these treatments attempt to treat the disease by increasing or decreasing the amount of particular gene expressed or the extent of the function of particular physiologically active factor developed, or by producing the physiologically active substance coded by the introduced nucleic acid. In most of such treatment, a vector (an agent for introduction) is used as a means for increasing the efficiency of nucleic acid introduction into the cell.

One typical vector is a viral vector, wherein infectivity of the virus inherent to the virus is utilized. Examples of such viral vectors include retrovirus vector and adenovirus vector, and exemplary applications of such viral vector are introduction of adenosine deaminase gene which is an attempt to treat adenosine deaminase deficiency by gene therapy (Blaese, R.M. et al., Science, Vol.270, 475 (1995)), and introduction of p53 gene for cancer treatment (Swisher, S.G. et al., J.Natl.Cancer Inst., Vol.91, 763 (1999)). A viral vector, however, is associated with a considerable risk in terms of its safety despite its excellent efficiency in introducing the nucleic acid to the cell. To be more specific, a viral vector is known to suffer from the risk of emergence of the wild type virus (pathogenic virus) and the risk of inducing a serious immune response by its high antigenicity. In addition, the process of production of a viral vector is an extremely complicated, and production of such viral vector in a commercial scale is generally difficult.

Another typical vector is a liposome vector. This vector utilizes the phenomenon that a charged liposome becomes attached to the cell and the liposome then becomes incorporated in the cell, and the liposome vectors known in the art include liposomes having a nucleic acid incorporated therein, and the liposomes aggregated around a nucleic acid forming a mass. Exemplary applications of the liposome having a nucleic acid incorporated therein include introduction of interferon β gene for treating brain tumor (M. Mizuno et al., Cancer Res., Vol.50, 7826 (1990)), and exemplary use of the method wherein a mass is formed by attaching liposomes around the nucleic acid include introduction of gene into a cultivated cell, which is frequently conducted in cell engineering experiment (Felgner, P.L. et al., Proc.Natl.Acad.Sci.U.S.A., Vol.84, 7413 (1987)). When the liposome vector is the one mainly comprising a natural phospholipid, the liposome vector is by far superior to the viral vector in view of safety. Such liposome vector, however, suffer from the problems of the complicated process of vector production, the complicated process of producing the complex of the vector and the nucleic acid, and the low efficiency in introducing the nucleic acid in the cell. When the liposome is constituted from a synthetic lipid, efficiency of nucleic acid introduction into the cell and handling convenience will be improved. Such liposome vector, however, suffers toxicity development. Also, both liposome vectors need further improvements in the drug preparation because of the poor storageability of the complex of the liposome and the nucleic acid.

Another exemplary vector is a peptide vector, and an example of such peptide vector is polylysine and its modified form. This vector utilizes the nature that a positively charged peptide tends to electrostatically bind to the negatively charged nucleic acid, and also to a cell. It has been revealed from various studies that, when the polylysine is used alone as a peptide vector, the oligonucleotide which has been covalently bonded to the polylysine is introduced in the cell (Lemaitre, M. et al., Proc.Natl.Acad.Sci.U.S.A., Vol.84, 648 (1987)). However, it has also been revealed that modification of the polylysine with a sugar, a glycoprotein, a phospholipid, or the like is required to substantially introduce the oligonucleotide or the plasmid that had been electrostatically bonded to the polylysine in a cell (Wu, G.Y. et al., J.Biol.Chem., Vol.262, 4429 (1987), Zhou, X. et al., Biochim.Biopys.Acta, Vol.1065, 8 (1991), Liang, W.W. et al., Biochim.Biopys.Acta, Vol.1279, 227 (1996)). It should also be noted that the unmodified polylysine exhibits significant toxicity when administered into the body of an animal.

In the meanwhile, an amphipathic basic peptide having α-helix structure has been shown to be a peptide which can be used alone as a vector for the nucleic acid since it exhibits high efficiency in introducing the nucleic acid due to its structural characteristics (Niidome, T. et al., J.Biol.Chem., Vol.272, 15307 (1997)). This peptide, however, suffers from the drawback that, in the α-helix structural model by Edmundson wheel plots, it exhibits typical two sided structure constituted from the hydrophobic side and the charged side (hydrophilic side), and when the proportion of the hydrophobic side is increased in order to maintain the ability of introducing the nucleic acid into the cell, such increase in the proportion of the hydrophobic side invites decrease of solubility in water. In such a case, if the proportion of the hydrophobic side were reduced in order to improve the solubility in water, ability of introducing the nucleic acid into the cell will be compensated. In addition, the proportion of the hydrophilic side, which is the charged surface of the peptide, is small, and hydrophilicity of the peptide is lost once the nucleic acid has become electrostatically bound to the peptide, and the complex of the peptide and the nucleic acid becomes hardly soluble. As a consequence, the peptide suffers from the drawback that aggregates are likely to be formed, and such aggregate formation is a substantial problem. Furthermore, the peptide will be highly toxic when it is administered to the interior of an animal due to the high tendency of the aggregate formation in serum.

As described above, despite the excellent general handling convenience of the peptide vector that it is capable of forming a complex merely by mixing with the nucleic acid, the peptide vector suffers from the drawback that it has a high tendency of forming aggregates especially in blood, and in addition, that the complex of the peptide vector and the nucleic acid exhibits a low solubility, and hence, a high toxicity. Therefore, the peptide vector has drawbacks for practical use.

In addition, all of the above-described vectors had no selectivity for the cell to which it is to be introduced, and the nucleic acid was introduced not only to the cell wherein the introduction of the nucleic acid was intended but also to the cell wherein the introduction of the nucleic acid was not at all intended. Such non-selective introduction has been associated with the risk of developing side effects.

By the way, phosphatidyl serine and phosphatidyl ethanolamine are aminophospholipids which are constituents of the lipid bilayer constituting the cell surface layer, and these aminophospholipids are phospholipids whose ratio of the content in the outer layer to the content in the inner layer of the lipid bilayer varies according to the conditions of the cell. To be more specific, phosphatidyl serine and phosphatidyl ethanolamine are phospholipids whose content in the outer layer of the lipid bilayer of the cell membrane increases in relation to the content in the inner layer when the cell receives some stimulus as typically found in the cell in the site proceeding blood coagulation reaction (Alan J. Schroit et al., Biochim. Biophys. Acta, Vol. 1071, 313 (1991)). Proportion of these phospholipids in the outer layer of the lipid bilayer are also believed to increase in the cells at the site where inflammation or cell activation and/or injury, apoptosis, or other so called "immunoresponsive" reaction caused by immunocompetent cell has taken place, the site where cells have become malignantly transformed through the progress of abnormal cell division, the site where the cells constituting the blood vessel have been injured by blood coagulation or by the progress of arterial sclerosis, the site where a cytotoxic reaction induced by an active enzyme is in progress, and the site where cell activation and/or cell injury by protease is in progress, and to be more specific, in the injured, denatured, or activated cell, namely, in the so called "abnormal" cell. Phosphatidyl serine is also known to be a phospholipid which is found in the granule, and which becomes translocated to the cell surface as a content of the granule in the course of degranulation in the cell (for example, mast cell and basophil) experiencing an allergic reaction (degranulation) caused by the binding of an allergen to the IgE antibody on the cell surface (Martin, S. et al., Int.Arch.Allergy and Immunol., Vol.123, 249 (2000)).

It is also to be noted that recent studies report that, even in the case of a cell which has been administered with an apoptosis-inducing substance (for example, an anticancer drug) or a cell which has been irradiated with radiation, and even if the cell had experienced signal transduction wherein p53 or other apoptosis-related gene had been involved, apoptosis does not take place in the case of a drug resistant cell (anticancer drug-resistant cancer cell etc.), and the drug resistant cell survives after DNA repair, and in the course of such DNA repair, phosphatidyl serine is translocated to the cell surface layer (Geske, FJ. et al., Cell Death Differ., Vol.8, 182 (2001)).

An object of the present invention is to provide a novel peptide which is highly safe, which exhibits excellent selectivity, which can be readily produced into a complex with a nucleic acid, which exhibits sufficient solubility and handling convenience, and which can be produced into a vector exhibiting high efficiency of introducing a nucleic acid into a cell.

### DISCLOSURE OF THE INVENTION

The inventors of the present invention have made an extensive study to solve the problems as described above, and found out that a peptide having affinity for a particular phospholipid can serve a vector for a nucleic acid, and such vector exhibits high efficiency in introducing the nucleic acid to the cell, low toxicity, and excellent solubility. It has also been found out that the peptide having particular structural characteristics defined by its amino acid sequence exhibits high tendency to be collected toward particular phospholipid, and hence, high selectivity in introducing the nucleic acid to the particular cell. The novel peptide has been invented on the bases of such findings.

The present invention provides the peptide as described in the following (1) to (8); a vector for gene therapy containing such peptide; a composition for gene therapy containing peptides and plasmids inserted with herpes simplex virus thymidine kinase gene; a method for treating or preventing cancer, tumor, arterial sclerosis, restenosis after percutaneous transluminal coronary angioplasty, or GVHD; and a method for delivering into a cell a substance that binds to such peptide by using such peptide.
(1) A peptide including a sequence comprising 18 amino acids, wherein
   said sequence of 18 amino acids is constituted from four sides comprising alternately arranged two hydrophobic sides and two hydrophilic sides in α-helix structural model depicted by Edmundson wheel plots,
   one of said hydrophobic sides comprises 5 to 7 amino acids and 80 mole% or more of this side comprises hydrophobic amino acids,
   one of said hydrophilic sides comprises 5 to 6 amino acids, and 80 mole% or more of this side comprises hydrophilic amino acids, and 50 mole% or more of this side comprises an amino acid selected from arginine and lysine,
   the other side of said hydrophobic sides comprises 2 to 4 hydrophobic amino acids, and
   the other side of said hydrophilic sides comprises 3 to 5 amino acids, and 80 mole% or more of this side comprises hydrophilic amino acids.
(2) A peptide according to (1) wherein said peptide comprises 20 or more amino acids in total; opposite ends of the peptide constitutes N and C terminals; and any 18 consecutive amino acids in the peptide excluding the amino acids at opposite ends constitutes said sequence of 18 amino acids.
(3) A peptide according to (1) or (2) wherein the amino acids at the N and C terminals are a hydrophilic amino acid.
(4) A peptide according to any one of (1) to (3) wherein any sequence comprising 18 consecutive amino acids in the following amino acid sequence constitutes said sequence of 18 amino acids
   X2-X3-X4-X5-X6-X7-X8-X9-X10-X11-X12-X13-X14-X15-X16- X17-X18-X19-X20-X21-X22-X23-X24-X25-X26-X27-X28-X29- X30-X31-X32-X33-X34-X35-X36,
   provided that,
   in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
   X3, X10, X12, X21, X28, and X30 are independently a member selected from a hydrophobic amino acid, a neutral hydrophilic amino acid and a basic hydrophilic amino acid,
   in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
   X6, X17, X24, and X35 are independently a hydrophobic amino acid, and
   X7, X14, X18, X25, X32, and X36 are independently a neutral hydrophilic amino acid or a basic hydrophilic amino acid.
(5) A peptide according to any one of (1) to (4) wherein said peptide comprises the following amino acid sequence:
   X1-X2-X3-X4-X5-X6-X7-X8-X9-X10-X11-X12-X13-X14-X15- X16-X17-X18-X19-X20-X21-X22-X23-X24-X25-X26-X27-X28- X29-X30-X31-X32-X33-X34-X35-X36-X37,
   provided that
   X1 and X37 are independently a hydrophilic amino acid,
   in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
   X3, X10, X12, X21, X28 and X30 are independently a member selected from a hydrophobic amino acid, a neutral hydrophilic amino acid, and basic hydrophilic amino acid,
   in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
   X6, X17, X24, and X35 are independently a hydrophobic amino acid, and
   X7, X14, X18, X25, X32, and X36 are independently a neutral hydrophilic amino acid or a basic hydrophilic amino acid; and wherein,
   the amino acids of from X2 to X36 may include deletion, addition, insertion, or substitution as long as at least said sequence of 18 amino acids is conserved in consecutive form.
(6) A peptide according to (5) wherein X1 to X37 are the following amino acids:
   X1 is threonine,
   X37 is serine,
   X2, X5, X9, X20, X23, and X27 are independently arginine or lysine,
   X3 and X21 are independently a member selected from tyrosine, phenylalanine, serine and arginine,
   X4, X17, X22, and X35 are independently leucine,
   X6, X15, X24, and X33 are independently leucine or isoleucine,
   X7, X13, X25, and X31 are independently histidine or arginine,
   X8 and X26 are independently proline,
   X10 and X28 are independently a member selected from serine, arginine, and leucine,
   X11 and X29 are independently tryptophan or leucine,
   X12 and X30 are independently a member selected from valine, leucine and serine,
   X14 and X32 are independently a member selected from glutamine, asparagine and arginine,
   X16 and X34 are independently alanine or arginine,
   X18 is a member selected from arginine, lysine and serine,
   X19 is leucine or threonines, and
   X36 is arginine or serine; and wherein
   the amino acids of from X2 to X36 may include deletion, addition, insertion, or substitution as long as at least said sequence of 18 amino acids is conserved in consecutive form.
(7) A peptide having the amino acid sequence of any one of SEQ ID NO:1 to SEQ ID NO:24.
(8) A peptide of SEQ ID NO:16 or SEQ ID NO:19.
(9) A vector for gene therapy containing the peptide of any one of claims (1) to (8).
(10) A composition for gene therapy containing the peptide of any one of (1) to (8) and plasmids including herpes simplex virus thymidine kinase gene.
(11) A composition for gene therapy according to (10) further comprising ganciclovir or acyclovir, said composition being used for treating or preventing cancer, tumor, arterial sclerosis, restenosis after percutaneous transluminal coronary angioplasty, or GVHD.
(12) A method for treating or preventing cancer, tumor, arterial sclerosis, restenosis after percutaneous transluminal coronary angioplasty, or GVHD characterized in that said method comprises administering ganciclovir or acyclovir simultaneously with or after administering the composition for gene therapy of (10), and continuing the administration of the ganciclovir or acyclovir at least for another 5 days.
(13) A method for delivering a substance which binds to the peptide of any one of (1) to (8) into a cell by using the peptide as the carrier.

### BRIEF DESCRIPTION OF THE DRAWINGS

FIGS. 1(A), 1(B), and 1(C) are schematic views showing α-helix structural model by Edmundson wheel plots. (In FIGS., the amino acids surrounded by square are hydrophobic amino acids, the amino acids surrounded by circle are basic hydrophilic amino acids, and the amino acids not surrounded by any of these are neutral hydrophilic amino acids. The same also applies to other drawings.)
FIG. 2 is a view showing an exemplary four sided structure of the sequence of 18 amino acids in the peptide of the present invention.
FIGS. 3(A) and 3(B) are views showing an exemplary four sided structure of the peptide of the present invention with the amino acids allocated to respective sides in a different manner.
FIG. 4(a) shows (C1), and FIG. 4(b) shows (C2). (C1) and (C2) are views showing an exemplary four sided structure of the peptide of the present invention with the amino acids allocated to respective sides in a different manner.
FIG. 5(A) and FIG. 5(B) are views showing an exemplary four sided structure of the peptide of the present invention including the sequence of 18 amino acids.
FIG. 6(a) shows (C), and FIG. 6(b) shows (D). (C) and (D) are views showing an exemplary four sided structure of the peptide of the present invention including the sequence of 18 amino acids.
FIG. 7(a) is a view showing mean residue ellipticity in CD spectroscopy under SDS(-) conditions. FIG. 7(b) is a view showing mean residue ellipticity in CD spectroscopy under SDS(+) conditions.
FIG. 8 (a) is a view showing mean residue ellipticity in CD spectroscopy under SDS(-) conditions. FIG. 8(b) is a view showing mean residue ellipticity in CD spectroscopy under SDS(+) conditions.
FIG. 9(a) is a view showing mean residue ellipticity in CD spectroscopy under SDS(-) conditions. FIG. 9(b) is a view showing mean residue ellipticity in CD spectroscopy under SDS(+) conditions.
FIG. 10 is an electropherogram of the mixture of the peptide of SEQ ID NO:1 and an oligonucleotide.
FIG. 11 is an electropherogram of the mixture of the peptide of SEQ ID NO:1 and a plasmid.
FIG. 12 is a view showing how a plasmid was introduced in the cell in the presence and absence of the peptide of SEQ ID NO:1 by using the luciferase activity expressed by the plasmid for the index.
FIG. 13 is a view showing increase of GCV sensitivity when a plasmid including HSV-tk gene was introduced in a cell by using the peptide of SEQ ID NO:16.
FIG. 14 is a view showing the ability of the peptide of SEQ ID NO:1 for introducing a plasmid in a cell before and after storing the complex of such peptide with the plasmid at 4°C by using the luciferase activity expressed by the plasmid for the index.
FIG. 15 is an electropherogram of an oligonucleotide after treating the mixture of the peptide of SEQ ID NO:1 and the oligonucleotide with a nuclease.
FIG. 16 is an electropherogram of a plasmid after treating the mixture of the peptide of SEQ ID NO:16 and the plasmid with a nuclease. Control is the plasmid which has not been treated by the nuclease.
FIG. 17 is a view showing the specific affinity of the peptide of SEQ ID NO:1 for phosphatidyl serine which has been measured by using Biacore 2000.
FIG. 18 is a view showing the measurements obtained by using Biacore 2000. The measurements indicate that the peptide of SEQ ID NO:25 has no affinity for either phosphatidyl serine or phosphatidyl choline.
FIG. 19 is a view showing the results of flow cytometry showing that, when a cell is stimulated for degranulation, phosphatidyl serine is translocated to the surface of the cell.
FIG. 20 is a view showing that the peptide of SEQ ID NO:16 introduces a larger amount of gene into the cell which has undergone degranulation with the phosphatidyl serine translocated to its surface.
FIG. 21 is a view showing that the peptide of SEQ ID NO:16 is capable of introducing a plasmid into the cancer cell that had been transplanted in a mouse, by using the luciferase activity expressed by the plasmid for the index.
FIG. 22 is a view showing that survival period of a mouse can be extended by introducing the plasmid containing HSV-tk gene in the cancer cell that had been transplanted in a mouse by using the peptide of SEQ ID NO:16, and thereafter administering GCV.

### BEST MODE FOR CARRYING OUT THE INVENTION

Next, the present invention is described in further detail.

The peptide according to the first aspect of the present invention is a peptide including an amino acid sequence comprising 18 amino acids, wherein said sequence of 18 amino acids is constituted from four sides comprising alternately arranged two hydrophobic sides (side A and side C) and two hydrophilic sides (side B and side D) in α-helix structural model depicted by Edmundson wheel plots (Edmundson, A.B. et al., Biophys.J., 7, Vol. 121 (1967)), and at least one side (side B) of the two hydrophilic sides (side B and side D) is a positively charged side.

Next, the amino acid sequence comprising 18 amino acids which is a structure critical in the peptide of the present invention is described by referring to the drawings.

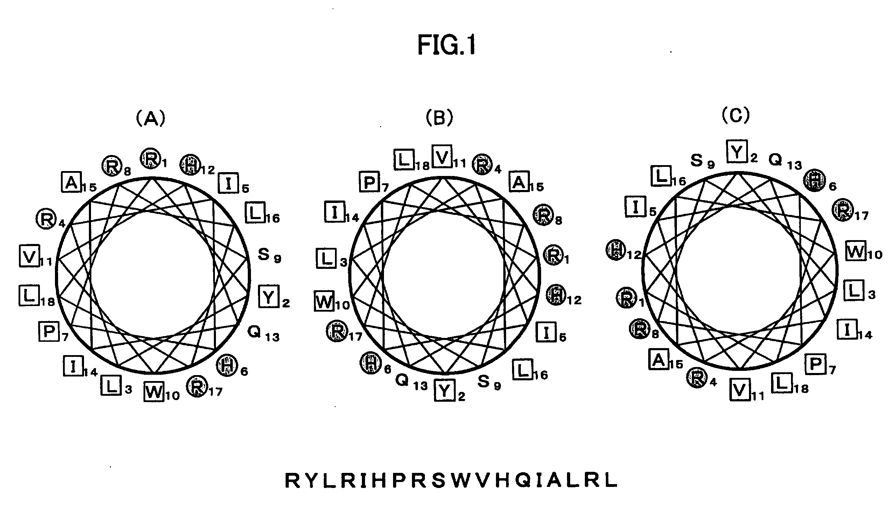

Edmundson wheel plot is a model which shows position of the amino acids in relation the central axis of the α-helix, and this plot is depicted so that the wheel is completed by 18 amino acids and the 19th amino acid comes to the same position as the 1st amino acid. In the model depicted by this method, the first amino acid located at the starting point is typically depicted at the position of 12 o'clock in a clock. There is, however, no difference in the relative location of the amino acids in the plot if the plotting were started from a different position in the drawing as long as the amino acid sequence is the same. For example, (A), (B), and (C) are essentially identical in FIG. 1.

In the present invention, the "side" designates an area of the model constituted by consecutive and adjacent amino acids. The number of amino acids constituting each side may be one or more, and preferably, each side may comprise two or more amino acids.

"Consecutive and adjacent" designates, for example, the positional relation how the 1st and the 12th amino acids, or four amino acids, namely, the 15th, the 8th, the 1st, and the 12th amino acids are located in FIG. 1. In contrast, the 1st and the 10th amino acids are not located in consecutive manner. The 1st and the 5th amino acids are also not located in "consecutive and adjacent" manner, while the 1st and 5th amino acids may constitute the same side together with the adjoining 12th amino acid.

The "hydrophobic side" is a side which includes a substantial number of hydrophobic amino acids. The hydrophobic amino acid is not particularly limited as long as it is substantially hydrophobic, and the hydrophobic amino acid may be a natural hydrophobic amino acid, or a modified or synthetic amino acid having characteristic features nearly equivalent to those of the natural amino acid.

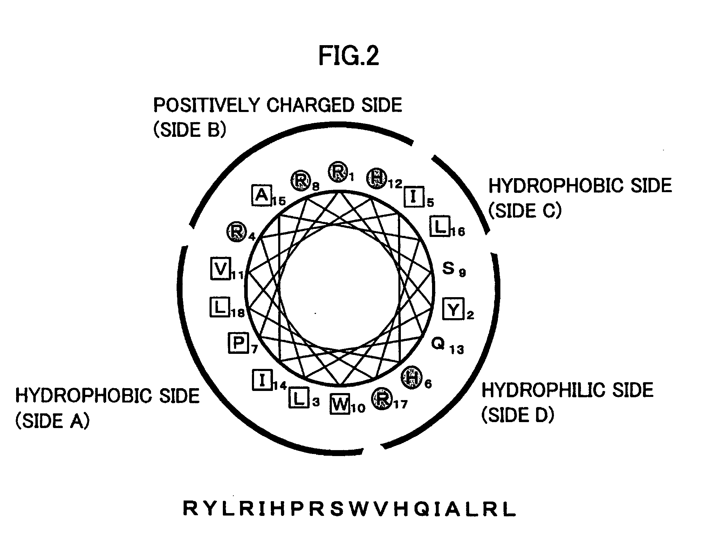

The peptide of the present invention may preferably include 80 mole% or more of hydrophobic amino acids in the hydrophobic side, and more preferably, no acidic hydrophilic amino acid (aspartic acid and glutamic acid) in the hydrophobic side since the acidic hydrophilic amino acid interferes with the electrostatic binding formed between the positively charged moiety of the peptide and the negatively charged moiety of the nucleic acid. The hydrophobic amino acids are preferably those selected from leucine, isoleucine, valine, tryptophan, proline, tyrosine, alanine, phenylalanine, methionine, cysteine, and glycine. One side (side A) of the hydrophobic sides may preferably comprise 5 to 7 amino acids. The other hydrophobic side (side C) may preferably comprise 2 to 4 amino acids. It is particularly preferable that side C comprises solely from hydrophobic amino acids. Typical hydrophobic sides (side A and side C) are shown in FIG. 2.

The "hydrophilic side" is a side which includes a substantial number of hydrophilic amino acids. The hydrophilic amino acid is not particularly limited as long as it is substantially hydrophilic, and the hydrophilic amino acid may be a natural hydrophilic amino acid, or a modified or synthetic amino acid having characteristic features nearly equivalent to those of the natural amino acid.

The "positively charged side" is a "hydrophilic side" which includes a considerable number of substantially positively charged hydrophilic amino acids. The hydrophilic amino acid may be a substantially positively charged, natural hydrophilic amino acid, or a modified or synthetic amino acid having characteristic features nearly equivalent to those of the natural amino acid.

The peptide of the present invention may preferably contain 80 mole% or more of hydrophilic amino acids in the hydrophilic side, and the hydrophilic amino acids are preferably those selected from asparagine, glutamine, threonine, serine, arginine, histidine, lysine, aspartic acid, and glutamic acid. It is more preferable that the hydrophilic amino acids are those other than acidic hydrophilic amino acids, namely, those selected from asparagine, glutamine, threonine, serine, arginine, histidine, and lysine since the acidic hydrophilic amino acid interferes with the electrostatic binding formed between the positively charged moiety of the peptide and the negatively charged moiety of the nucleic acid.

One side (side B) of the hydrophilic sides is preferably a positively charged side comprising 5 to 6 amino acids. More preferably, 50 mole% or more of the amino acids constituting this side are selected from lysine and arginine. The other hydrophilic side (side D) may preferably comprise 3 to 5 amino acids. Typical hydrophilic side (side D) and positively charged side (side B) are shown in FIG. 2.

It is to be noted that the "mole%" used herein designates the ratio of the number of hydrophobic amino acids in the hydrophobic side to the number of amino acids constituting the hydrophobic side, the ratio of the number of hydrophilic amino acids in the hydrophilic side to the number of amino acids constituting the hydrophilic side, or the ratio of the number of amino acids selected from lysine and arginine in the positively charged side to the number of amino acids constituting the positively charged side.

The four sided structure comprising the alternately arranged two hydrophobic sides and two hydrophilic sides (wherein at least one of the hydrophilic sides is a positively charged side) which is the characteristic feature of the peptide of the present invention is a structure wherein, when the 18 amino acids shown in the model is divided into the sides in accordance with the definition as described above, two hydrophobic sides (side A and side C) and two hydrophilic sides (side B and side D, wherein at least side B is a positively charged side) are alternately arranged to constitute the four side. It is to be noted that, when a hydrophobic side is directly juxtaposed to another hydrophobic side, these sides are not regarded as two hydrophobic sides but as one integrated hydrophobic side; and when a hydrophilic side is directly juxtaposed to another hydrophilic side, these sides are regarded not as two hydrophilic sides but one integral hydrophilic side; and when a positively charged side is directly juxtaposed to another positively charged side, these sides are regarded not as two positively charged sides but as one integral positively charged side. In other words, in the four sided structure, the hydrophobic side is not adjacent to another hydrophobic side, the hydrophilic side is not adjacent to another hydrophilic side, and the positively charged side is not adjacent to another positively charged side.

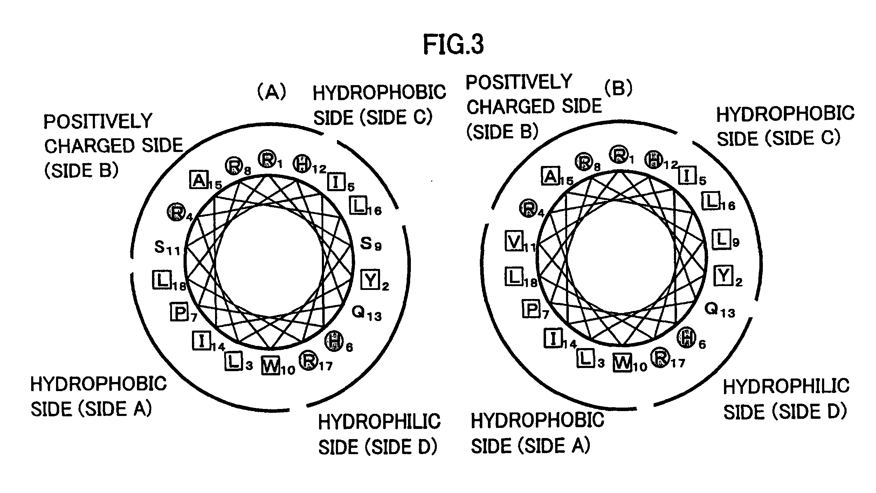

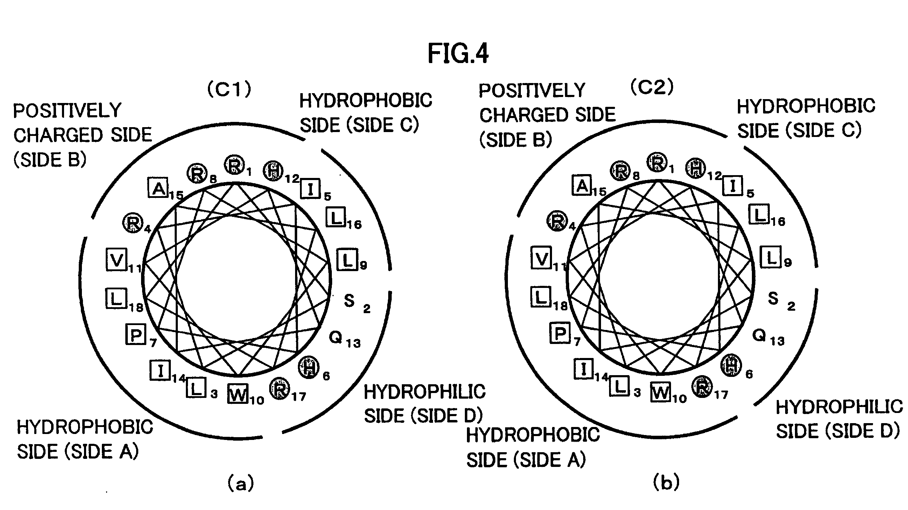

"The structure wherein two hydrophobic sides and two hydrophilic sides are alternately arranged to constitute the four side" is not particularly limited as long as the four sides are arranged [side A → side B → side C → side D (→ side A)] or [side A → side D → side C → side B (→ side A)] in clockwise in the α-helix structural model of Edmundson wheel plot, and the function is retained. The preferable sequence is the one wherein sides are arranged [side A → side B → side C → side D (→ side A)] in clockwise. It is to be noted that the four sided structure is not limited for its method how the amino acids are divided, namely, how the 18 amino acids are allocated to each of four sides as long as each side retains its character. The amino acids are preferably allocated on the bases of the amino acid sequence such that side A comprises 5 to 7 amino acids, side B comprises 5 to 6 amino acids, and side C comprises 2 to 4 amino acids, and side D comprises 3 to 5 amino acids. Typical three sequences wherein the amino acid sequence is allocated to each side in a different manner are shown as sequence A to C (C1 and C2) in FIGS. 3 and 4. It is to be noted that the amino acid sequence of C1 and C2 is completely the same, and the different allocations are both within the scope of the definition as described above. The allocation of amino acids in A to C (C1 and C2) are as described below.

| | Hydrophobic side | Hydrophilic side | Hydrophobic side | Hydrophilic side |
|---|---|---|---|---|
| | (Side A) | (Side B) | (Side C) | (Side D) |
| A | 5 | 6 | 2 | 5 |
| B | 6 | 5 | 4 | 3 |
| C1 | 6 | 5 | 3 | 4 |
| C2 | 7 | 5 | 3 | 3 |

Since the peptide of the present invention contains the amino acid sequence comprising 18 amino acids exhibiting the four sided structure as its characteristic feature, the peptide has excellent solubility in water. The peptide also has a characteristic feature that it is capable of forming a complex with a nucleic acid without forming aggregates of the complex which may cause a substantial problem.

In contrast to the peptide vector having an α-helix structure which has been described in the section of "Prior Art", in the case the peptide of the present invention, a plurality of hydrophobic sides are formed when the peptide takes α-helix structure, and therefore, the peptide has high ability of introducing the nucleic acid into the cell even when the proportion of the hydrophobic sides is reduced for the purpose of improving the solubility in water. A plurality of hydrophilic sides are also formed simultaneously, and accordingly, proportion of the hydrophilic sides is higher compared to the peptide of two sided structure, and the hydrophilicity of the peptide does not completely disappear even when the nucleic acid becomes electrostatically bonded to the at least one positively charged side of the peptide. Solubility in water of the complex of the peptide and the nucleic acid is thereby maintained, and as a consequence, formation of troublesome aggregate masses is prevented. As described above, if the peptide were to have a high nucleic acid-introducing ability simultaneously with an excellent solubility in water, a plurality of hydrophobic sides and a plurality of hydrophilic sides (of which at least one side is the positively charged side) need to be formed when the peptide takes α-helix structure. It should also be noted that the peptide is not limited for its number of sides as long as two or more hydrophobic sides and two or more hydrophilic sides are formed, and the function as a peptide vector is maintained. However, the peptide may preferably have two hydrophobic and two hydrophilic sides, and in particular, at least one side of the two or more hydrophilic sides is preferably a side rich in neutral hydrophilic amino acids (namely, a side with no substantial charge), since such side will not become bonded to the nucleic acid and water solubility of such side will substantially be fully maintained.

The peptide of the present invention is by no means limited for its length of the amino acid sequence as long as its function is retained. The peptide, however, is preferably the one having a total amino acid residue number of 20 or more, more preferably 25 or more, and most preferably 30 or more. The peptide may preferably have a total amino acid residue number of up to 100, more preferably up to 50, and most preferably up to 40.

The peptide of the present invention includes at least one amino acid sequence of 18 consecutive amino acids starting from any amino acid. Preferably, the peptide of the present invention is a peptide containing at least two independent amino acid sequences of 18 consecutive amino acids starting from any amino acid; and/or at least two overlapping amino acid sequences of 18 consecutive amino acids. More preferably, the peptide of the present invention is a peptide wherein any consecutive 18 amino acids excluding the amino acids at the opposite ends represents the amino acid sequence of 18 amino acids exhibiting the four sided structure of the present invention, that is, a peptide wherein all of the overlapping amino acid sequences comprising 18 consecutive amino acids excluding the amino acids at the opposite ends exhibits the four sided structure of the present invention.

"The amino acid sequence comprising 18 amino acids" used herein designates an amino acid sequence comprising 18 amino acids wherein side A, side B, side C, and side D are arranged in clockwise direction in the α-helix model by Edmundson wheel plot (hereinafter referred to as "the four sided structure of the present invention") (Such sequence is hereinafter referred to as "the amino acid sequence comprising 18 amino acids exhibiting the four sided structure of the present invention".)

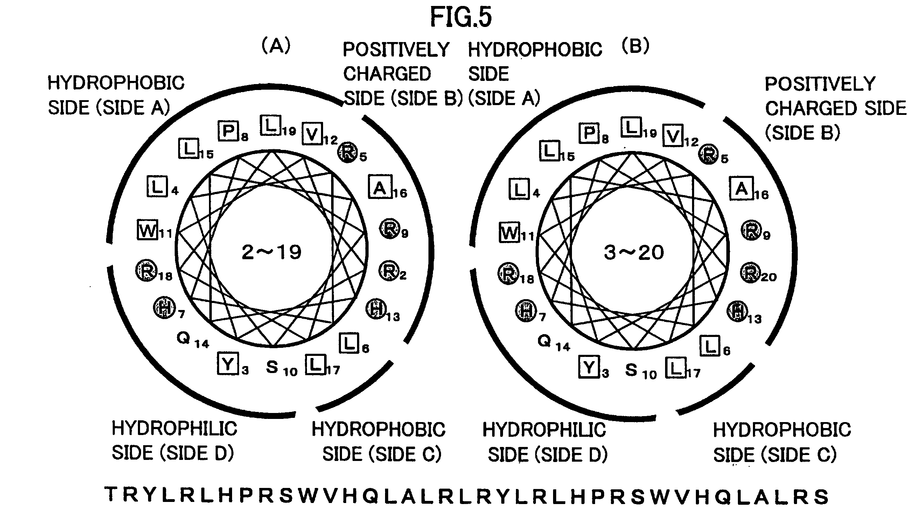

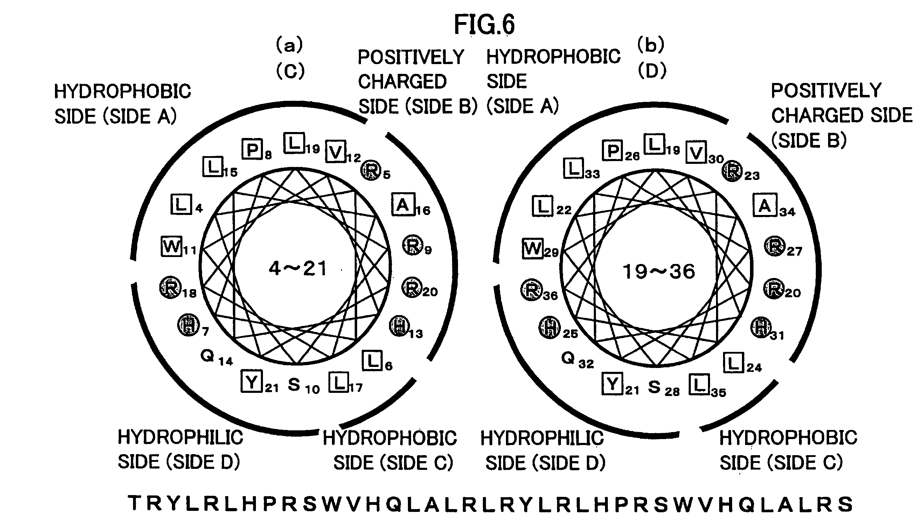

"The amino acid sequence comprising any consecutive 18 amino acids excluding the amino acids at opposite ends" means, for example, any amino acid sequence in a peptide comprising N amino acids (wherein N represents a number of 20 or more) comprising 2nd to 19th, 3rd to 20th, 4th to 21st, or in a similar manner, (N-18)th to (N-1)th amino acids. "All of the overlapping amino acid sequences comprising 18 consecutive amino acids excluding the amino acids at the opposite ends exhibits the four sided structure of the present invention" means that all of the above-mentioned sequences comprising 2nd to 19th, 3rd to 20th, 4th to 21st, or in a similar manner, (N-18)th to (N-1)th amino acids exhibit the four sided structure of the present invention. Examples of such amino acid sequence are shown in FIGS. 5 and 6 as sequences A to D in the α-helix model by Edmundson wheel plot, and the sequences shown are the sequences comprising 18 amino acids of from 2nd to 19th, from 3rd to 20th, from 4th to 21st, and from 19 to 36th amino acids in the peptide of SEQ ID NO:16. All of these sequences show the four sided structure of the present invention.

In view of further improving the solubility of the complex of the peptide and the substance which binds to the peptide, the peptide of the present invention is preferably the one wherein at least one end comprises a hydrophilic amino acid, and more preferably, the one wherein both ends comprise a hydrophilic amino acids. The "substance which binds to the peptide" used herein designates a physiologically active substance such as a nucleic acid.

The hydrophilic amino acid is not particularly limited as long as it is hydrophilic. The hydrophilic amino acid, however, is preferably the one other than acidic hydrophilic amino acid, and more preferably, a neutral hydrophilic amino acid, and most preferably threonine or serine.

Preferable examples of "the amino acid sequence of 18 amino acids exhibiting the four sided structure of the present invention" included in the peptide of the present invention are the amino acid sequences comprising any consecutive 18 amino acids in the following amino acid sequence:
X2-X3-X4-X5-X6-X7-X8-X9-X10-X11-X12-X13-X14-X15-X16- X17-X18-X19-X20-X21-X22-X23-X24-X25-X26-X27-X28-X29- X30-X31-X32-X33-X34-X35-X36,
provided that,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
X3, X10, X12, X21, X28, and X30 are independently a member selected from a hydrophobic amino acid, a neutral hydrophilic amino acid and a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid.

Preferably,
X8 and X26 are proline,
X4, X17, X22, and X35 are leucine,
X6, X11, X15, X24, X29, and X33 are independently a hydrophobic amino acid,
X12, X19, and X30 are independently a hydrophobic amino acid or a neutral hydrophilic amino acid,
X2, X5, X9, X20, X23, and X27 are independently a basic hydrophilic amino acid,
X13 and X31 are independently a basic hydrophilic amino acid or a neutral hydrophilic amino acid,
X16 and X34 are independently a hydrophobic amino acid or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 3 amino acid in the 5 amino acids are arginine or lysine,
X3, X10, X21, and X28 are independently a member selected from a hydrophobic amino acid, a neutral hydrophilic amino acid, and a basic hydrophilic amino acid, and
X7, X14, X18, X25, X32, and X36 are independently a neutral hydrophilic amino acid or a basic hydrophilic amino acid.

More preferably,
X2, X5, X9, X20, X23, and X27 are independently arginine or lysine,
X3 and X21 are independently a member selected from tyrosine, phenylalanine, serine, and arginine,
X4, X17, X22, and X35 are independently leucine,
X6, X15, X24, and X33 are independently leucine or isoleucine,
X7, X13, X25, and X31 are independently histidine or arginine,
X8 and X26 are independently proline,
X10 and X28 are independently a member selected from serine, arginine, and leucine,
X11 and X29 are independently tryptophan or leucine,
X12 and X30 are independently valine, leucine, or serine,
X14 and X32 are independently a member selected from glutamine, asparagine, and arginine,
X16 and X34 are independently alanine or arginine,
X18 is a member selected from arginine, lysine and serine,
X19 is leucine or threonine, and
X36 is arginine or serine.

It should be noted that, in the above description, a hydrophobic amino acid is an amino acid selected from leucine, isoleucine, valine, tryptophan, proline, tyrosine, alanine, cysteine, phenylalanine, methionine, and glycine; a basic hydrophilic amino acid is an amino acid selected from arginine, histidine, and lysine; and a neutral hydrophilic amino acid is an amino acid selected from asparagine, glutamine, threonine, and serine.

A preferable example of the peptide of the present invention is a peptide comprising the following amino acid sequence:
X1-X2-X3-X4-X5-X6-X7-X8-X9-X10-X11-X12-X13-X14-X15- X16-X17-X18-X19-X20-X21-X22-X23-X24-X25-X26-X27-X28- X29-X30-X31-X32-X33-X34-X35-X36-X37,
provided that
X1 and X37 are a hydrophilic amino acid,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
X3, X10, X12, X21, X28 and X30 are independently a member selected from a hydrophobic amino acid, a neutral hydrophilic amino acid, and basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid.

It is to be noted that in the amino acid sequence as described above, the amino acids of from X2 to X36 may include deletion, addition, insertion, or substitution as long as at least the amino acid sequence of 18 amino acids is conserved in consecutive form.

Preferably,
X1 and X37 are independently threonine or serine,
X8 and X26 are independently proline,
X4, X17, X22, and X35 are independently leucine,
X6, X11, X15, X24, X29, and X33 are independently a hydrophobic amino acid,
X12, X19, and X30 are independently a hydrophobic amino acid or a neutral hydrophilic amino acid,
X2, X5, X9, X20, X23, and X27 are independently a basic hydrophilic amino acid,
X13 and X31 are independently a basic hydrophilic amino acid or a neutral hydrophilic amino acid,
X16 and X34 are independently a hydrophobic amino acid or a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 3 amino acids in the 5 amino acids is arginine or lysine,
X3, X10, X21, and X28 are independently a member selected from a hydrophobic amino acid, a neutral hydrophilic amino acid, and a basic hydrophilic amino acid, and
X7, X14, X18, X25, X32, and X36 are independently a neutral hydrophilic amino acid or a basic hydrophilic amino acid.

It is to be noted that the amino acids of from X2 to X36 may include deletion, addition, insertion, or substitution as long as at least the amino acid sequence of 18 amino acids is conserved in consecutive form.

More preferably,
X1 is threonine,
X37 is serine,
X2, X5, X9, X20, X23, and X27 are independently arginine or lysine,
X3 and X21 are independently a member selected from tyrosine, phenylalanine, serine and arginine,
X4, X17, X22, and X35 are independently leucine,
X6, X15, X24, and X33 are independently leucine or isoleucine,
X7, X13, X25, and X31 are independently histidine or arginine,
X8 and X26 are independently proline,
X10 and X28 are independently a member selected from serine, arginine, and leucine,
X11 and X29 are independently tryptophan or leucine,
X12 and X30 are independently a member selected from valine, leucine and serine,
X14 and X32 are independently a member selected from glutamine, asparagine and arginine,
X16 and X34 are independently alanine or arginine,
X18 is a member selected from arginine, lysine and serine,
X19 is leucine or threonine, and
X36 is arginine or serine.

It is to be noted that in the amino acid sequence as described above, the amino acids of from X2 to X36 may include deletion, addition, insertion, or substitution as long as at least the amino acid sequence of 18 amino acids is conserved in consecutive form.

In the above description, the hydrophobic amino acid is an amino acid selected from leucine, isoleucine, valine, tryptophan, proline, tyrosine, alanine, cysteine, phenylalanine, methionine and glycine; the basic hydrophilic amino acid is an amino acid selected from arginine, histidine, and lysine; and the neutral hydrophilic amino acid is an amino acid selected from asparagine, glutamine, threonine, and serine.

It is to be noted that the amino acid sequences as mentioned above are only some embodiments of the peptide of the present invention, and the peptide of the present invention may be the amino acid sequences as mentioned above including deletion, addition, insertion, or replacement as desired as long as the peptide include "the amino acid sequence of 18 amino acids exhibiting the four sided structure of the present invention", and the peptide retains its function. If necessary, the peptide may also be modified by a molecule other than amino acid. For example, the peptide may be modified by a sugar chain, a lipid, or a high molecular weight compound in order to increase in vivo stability, and/or by a sugar chain, a lipid, or a high molecular weight compound in order to suppress the recognition of the peptide by an antigen presenting cell. To be more specific, the peptide may be modified with mannose, cholesterol, or polyethylene glycol, and such modified peptides are also within the scope of the peptide of the present invention.

The peptide according to the preferred embodiment of the present invention does not contain any acidic hydrophilic amino acid, and this feature is particularly useful when the peptide is to be modified. To be more specific, such peptide can be designed to include no acidic amino acid and to include carboxyl group only at its C terminal, and when the peptide is modified by utilizing a reaction depending on the carboxyl group, a site specific modification of the C terminal is enabled.

When the peptide of the present invention is site-specifically modified by utilizing thiol group in cysteine residue, the peptide may be designed so that only one cysteine is present in the amino acid sequence, and preferably, so that the cysteine is added at the N terminal or C terminal of peptide of the present invention. When the peptide of the present invention is designed so that only one lysine is present in the amino acid sequence, and preferably, so that the lysine is added at the N terminal or C terminal of the peptide of the present invention, selective modification of the peptide is enabled, for example, in the modification with polyethylene glycol.

By the way, amino acids are categorized by the type and nature of their side chain molecules, and the categorization which may serve an important index in elucidating higher order structure of the peptide is the categorization based on the polarity of the side chain molecule. To be more specific, the amino acids are categorized as described below.
(1) Hydrophobic amino acid: glycine, alanine, valine, leucine, isoleucine, methionine, phenylalanine, tyrosine, cysteine, tryptophan, and proline
(2) Acidic hydrophilic amino acid: aspartic acid, and glutamic acid
(3) Neutral hydrophilic amino acid: serine, threonine, glutamine, and asparagine
(4) Basic hydrophilic amino acid: arginine, lysine, and histidine

Accordingly, in the peptide of the present invention, amino acids which belong to the same category are mutually replaceable as long as the requirement that "the amino acid sequence of 18 amino acids exhibits the four sided structure of the present invention" is fulfilled. For example, isoleucine and leucine, valine and leucine, tyrosine and phenylalanine, tryptophan and leucine, asparagine and glutamine, serine and threonine, arginine and lysine, and histidine and lysine are mutually replaceable. However, in the case of histidine which is categorized as a member of basic hydrophilic amino acids, it is only weakly charged under particular conditions, for example, under the physiological conditions, and it shares the nature similar to that of a neutral hydrophilic amino acid, and therefore, histidine is not only replaceable with a basic hydrophilic amino acid, but also with a neutral hydrophilic amino acid.

In the meanwhile, the amino acids which belong to different categories are also mutually replaceable as long as the requirement that "the amino acid sequence of 18 amino acids exhibits the four sided structure of the present invention" is fulfilled. Typical examples of such replacements are as described below.
(1) Replacement between a hydrophobic amino acid (neutral) and a neutral hydrophilic amino acid (which is equivalent to replacement between arbitrary neutral amino acids)
   Examples: leucine and threonine; leucine and serine; valine and serine; and tyrosine and serine.
(2) Replacement between a hydrophobic amino acid (neutral) and a basic hydrophilic amino acid (which is a replacement between opposites, namely, hydrophobic/hydrophilic and neutral/basic amino acids, and which is equivalent to the replacement between any amino acids other than acidic hydrophilic amino acids)
   Examples: alanine and arginine; and tyrosine and arginine.
(3) Replacement between a neutral hydrophilic amino acid and a basic hydrophilic amino acid (which is equivalent to the replacement between any hydrophilic amino acids other than acidic hydrophilic amino acids)
   Examples: serine and arginine; and glutamine and arginine.

Furthermore, two or more of the amino acid replacements as described above may be combined as long as the requirement that "the amino acid sequence of 18 amino acids exhibits the four sided structure of the present invention" is fulfilled. An exemplary combination of the amino acid replacements between the amino acids of the same category is the replacement of isoleucine with leucine combined with the replacement of valine with leucine. An exemplary combination of the amino acid replacements between the amino acids of different categories is the replacement of tyrosine with serine combined with the replacement of serine with leucine. An exemplary combination of the amino acid replacement between the amino acids of the same category and the amino acid replacements between the amino acids of different categories is the replacement of isoleucine with leucine combined with the replacement of leucine with threonine. The number of the amino acid replacements that may be combined is not limited as long as the requirement that "the amino acid sequence of 18 amino acids exhibits the four sided structure of the present invention" is fulfilled. However, the number of amino acid replacements combined is preferably 3 or less per 18 amino acids.

For example, Examples 12 and 13 demonstrate that the peptide of the present invention can include amino acid replacements as long as the requirement that "the amino acid sequence of 18 amino acids exhibits the four sided structure of the present invention" is fulfilled.

A typical peptide of the present invention is a peptide having the amino acid sequence of any of SEQ ID NO:1 to SEQ ID NO:24, and the peptide of the present invention is preferably a peptide having the amino acid sequence of SEQ ID NO:16 or SEQ ID NO:19.

The peptide of the present invention is a peptide which has an ability of binding to a nucleic acid and an ability of introducing the nucleic acid to a cell, and which also has an affinity specific for phosphatidyl serine.

In the present specification, the peptide is not limited for its length of the amino acid sequence, and the peptide may be from the so-called dipeptide containing two amino acids to polypeptide containing 1,000 or more amino acids. The amino acids constituting the peptide may be either L- or D-amino acids, and may be amino acids other than typical amino acids or synthetic, modified amino acids as long as they substantially share common nature with the natural amino acids. Exemplary such amino acids include hydroxyproline, homoserine, and methylcysteine.

The "nucleic acid" used herein includes a nucleoside, a nucleotide, an oligonucleotide or a polynucleotide comprising two or more nucleotides, a DNA, an RNA, a derivative thereof, a modification thereof, and an analog thereof. The "nucleic acid derivative" includes a nucleic acid wherein some of the atoms constituting the nucleic acid have been replaced with other atoms. An example of such "nucleic acid derivative" is the PS form wherein one of the oxygen atoms in the phosphodiester bond moiety has been replaced with sulfur atom. The "modified nucleic acid" includes a nucleic acid wherein some of the atoms constituting the nucleic acid have been replaced with other atomic group or some of the atoms constituting the nucleic acid have other atomic group added thereto. Examples of such "modified nucleic acid" are the one wherein the carbon atom located at 2' position of the pentose moiety in the nucleic acid has methoxy group (-O-CH₃) added thereto, and those wherein the nucleic acid sequence has a sugar, a phospholipid, or polyethylene glycol added thereto in some part thereof. The "nucleic acid analog" include a molecule which has a backbone entirely different from that of a nucleic acid while the molecule retains the function expected for a nucleic acid. An example of such "nucleic acid analog" is a peptide nucleic acid (PNA). The "nucleic acid" used herein also includes a DNA or an RNA which is a polynucleotide that increases or decreases the amount of particular protein expressed in the body, or regulates the expression of the function of particular factor in the body; a derivative, a modification, or an analog of such DNA or RNA; a DNA or an RNA which is simultaneously combined a derivative, a modification, and an analog; and a mixture or chimera of such derivative, modification, and analog of the DNA or the RNA. The nucleic acid also includes a nucleoside, a nucleotide, an oligonucleotide or a polynucleotide comprising two or more nucleotides, a derivative thereof, a modification thereof, an analog thereof, one which is simultaneously combined a derivative, a modification, and an analog; and a mixture or chimera thereof.

Furthermore, the "nucleic acid" may be a single stranded or double or more stranded nucleic acid, and may be the one bound to a carrier. For example, the nucleic acid may be the DNA coding for a protein, a plasmid wherein an expression-regulating unit has been linked to such DNA, an antisense oligonucleotide, a double-stranded nucleic acid serving as a decoy (hereinafter referred to as decoy), an aptamer, or a ribozyme. The "particular protein" or the "particular factor" used herein designates a protein or a factor whose amount expressed is to be increased or decreased, or whose expression is to be regulated by the nucleic acid. Such protein and factor may be either the one found in a living body or the one not found in a living body.

The "ability of binding to a nucleic acid" can be assayed by subjecting a mixture of the nucleic acid and the peptide of the present invention to electrophoresis, and detecting the image of the stained nucleic acid. For example, when the nucleic acid is not electrophoresed and the stained image of the nucleic acid is not detected, or when the stained image detected is in the region where distance of the migration of the stained image is small compared to the stained image of the nucleic acid alone, the peptide can be determined to have "the ability of binding to a nucleic acid". Illustrative procedure of such assay will be described in Example 8.

The "ability of introducing a nucleic acid to a cell" can be measured by observing the cell under a fluorescence microscope using a fluorescent-labeled nucleic acid, or by using a plasmid which expresses a reporter gene and measuring the reporter protein expressed by the cell. The "ability of introducing a nucleic acid to a cell" can also be measured by using the pharmacological action resulting from the expression of the reporter protein for the index. Typical reporters include firefly luciferase, β-galactosidase, and HSV-tk, and illustrative procedure will be described in Example 9. When the amount of the firefly luciferase expressed by the cell is measured by such procedure, fluorescent count per 1 mg of protein per 1 second is measured. When the fluorescent count is 10,000 or more, the reporter gene is determined to have been introduced into the cell, and the peptide is determined to have the "ability of introducing a nucleic acid to a cell".

It is to be noted that, in the present invention, the "introduction in (in/into/to) the cell" designates the same situation as the "introduction into the interior of the cell".

In the present invention, "in the cell (or interior of the cell)" designates the units constituting the cell and their interior. For example, included in the "in the cell" are interior of the phospholipid bilayer constituting the contour of the cell, the space between the phospholipid bilayers, cytoplasm, organella, nucleus, and their interior.

"Specific affinity" means that the peptide of the present invention exhibits some specific interaction, for example, binding, formation of complex, mutual recognition of the molecule, tendency of moving in a particular direction or being collected in a particular direction, change of molecular configuration, or mutual reaction. For example, the peptide is determined to have a specific affinity if the peptide exhibits affinity in the presence of serum albumin.

Even if a peptide interacts with a particular substance, the interaction is generally nonspecific if the interaction disappears in the presence of other peptides or other proteins. To be more specific, a nonspecific interaction disappears in the presence of a large amount of albumin or other protein. Accordingly, a peptide which exhibits affinity for phosphatidyl serine in the absence of serum albumin but fails to exhibit affinity for phosphatidyl serine in the presence of serum albumin is nonspecific with regard to the interaction with phosphatidyl serine, and the peptide will exhibit no affinity for phosphatidyl serine in a living body.

On the other hand, when the interaction of the peptide with the particluar substance does not disappear in the presence of other peptides or other proteins, the interaction can be deemed specific, and the peptide can be regarded to have a "specific affinity".

The peptide according to the first aspect of the present invention has the ability of binding to a nucleic acid. The peptide of the present invention is not limited for its mode of binding to the nucleic acid, and the binding may be accomplished, for example, by electrostatic bonding, hydrophobic bonding, or covalent bonding. The peptide is preferably the one provided with the ability of forming a complex with the nucleic acid by substantial integration between the peptide and the nucleic acid without completely compromising any of other abilities of the peptide such as the ability of introducing the nucleic acid to a cell or the affinity for phosphatidyl serine in the presence of serum albumin, or the functions inherent to the nucleic acid.

The peptide of the present invention has ability of introducing the nucleic acid that has become bonded to the peptide into the interior of the cell. The peptide of the present invention exhibits a fluorescent count per 1 mg of protein per 1 second of at least 10,000 in the measurement of Example 9. Preferably, the peptide of the present invention is a peptide which exhibits the fluorescent count of at least 100,000, and more preferably, a peptide exhibiting the fluorescent count of at least 1,000,000.

Preferably, the peptide of the present invention is also a peptide which has ability of introducing the nucleic acid without causing decomposition of the nucleic acid and with the desired function of the nucleic acid maintained.

Since the peptide of the present invention is provided with the features as described above, the nucleic acid introduced by using the peptide is capable of exerting its desired functions in the cell once it is introduced in the cell. For example, the exogenous gene inserted in the plasmid may become expressed in the cell to produce the desired protein, or the antisense oligonucleotide, decoy, aptamer, ribozyme, or the like may suppress production of a particular physiologically active substance. It is to be noted that "the desired protein" not only includes the final active form of the protein but also the precursor for such final form of the protein. Examples 11 and 24 will illustrate embodiments wherein firefly luciferase gene that has been inserted in a plasmid is introduced in a cell by the peptide of the present invention, and after its transcription and translation, firefly luciferase is produced and accumulated in the cell. Examples 16 and 25 will illustrate embodiments wherein thymidine kinase (hereinafter abbreviated as HSV-tk) gene from herpes simplex virus that has been inserted in the plasmid is introduced in a tumor cell by the peptide of the present invention, and after its transcription and translation, HSV-tk is produced and accumulated in the tumor cell to thereby enhance ganciclovir sensitivity of the tumor cell and exert pharmacological action (anti-tumor action).

Furthermore, the peptide according to a preferred embodiment of the present invention is a peptide which exhibits specific affinity for phosphatidyl serine in the presence serum albumin, and which does not exhibits any affinity for phospholipids other than phosphatidyl serine, for example, phosphatidyl choline.

The interaction between the peptide according to the preferred embodiment of the present invention which has no affinity for a phospholipid other than phosphatidyl serine and the phosphatidyl serine, namely, the binding between the peptide of the invention and the phosphatidyl serine is not limited to the binding through charge or the binding through non-specific binding, and the binding may also be the binding enabled by the recognition of the molecular structure of the phosphatidyl serine by the peptide. This is demonstrated, for example, in Example 20 and Example 21.

As described above, phosphatidyl serine is the phospholipid which is included as a component constituting the lipid bilayer forming the surface layer of a cell, and' the proportion of the phosphatidyl serine found in the outer layer and the inner layer of the lipid bilayer varies depending on the condition of the cell. To be more specific, phosphatidyl serine is a phospholipid which is believed to increase its proportion in the outer layer of the lipid bilayer in an abnormal cell such as a cell in the inflammatory lesion wherein the cell has been injured, denatured or activated. Therefore, the peptide according to the first aspect of the present invention selectively binds to the abnormal cell, for example, the cell in the inflammatory lesion. Phosphatidyl serine is also a phospholipid which provides a "field" in a living body for the blood coagulation reaction to take place, or a mark when macrophage recognizes and eats an apoptotic cell. Therefore, the peptide according to the first aspect of the present invention selectively binds to the abnormal cell, for example, to the "field" in a living body where blood coagulation reaction is in progress.

The peptide according to the first aspect of the present invention is characterized by its ability of binding to a nucleic acid, its ability of introducing the nucleic acid to a cell, and its affinity for phosphatidyl serine in the presence of serum albumin.

The peptide according to the first aspect of the present invention is preferably a peptide which takes an irregular structure in an aqueous solution containing no solute or only an inorganic salt but which takes the α-helix structure in the presence of a particular substance. The "particular substance" used herein designates a substance which interacts with the peptide of the present invention to promote the peptide to take the α-helix structure, and an exemplary such substance is an amphipathic substance, for example, a surfactant such as sodium dodecyl sulfate (SDS) or a particular phospholipid such as phosphatidyl serine.

α-helix structure in the higher order structure of a peptide can be generally confirmed by measuring CD spectrum. To be more specific, when α-helix structure is present in the higher order structure of a peptide, mean residue ellipticity in the CD spectroscopy takes the form of "W" which is characteristic to the α-helix structure wherein local minimum is found in two wavelength regions, namely, in the region at the wavelength of 205 to 210 nm and the region at the wavelength of 220 to 225 nm. (See "Optical rotation of proteins" (Experimental methods in biological chemistry 6), Hamaguchi, H. et al., Japan Scientific Societies Press, 1979). It is to be noted that the proportion of the α-helix structure in the higher order structure of the peptide can be calculated by a predetermined calculation method from the mean residue ellipticity that had been measured. Typical examples of such calculation include the method of Chen et al. (Y.H. Chen et al., Biochemistry Vol.11, 4120 (1972)), the method of Yang et al. (J.T. Yang et al., Anal.Chem., Vol.91, 13 (1978)), and the method of Woody et al. (R.W. Woody et al., J.Mol.Biol., Vol. 242, 497 (1994)). The value calculated, however, varies by the method used for the calculation, and therefore, it is recommended that the method employed for the calculation is indicated together with the value calculated.

The peptide according to the preferred embodiment of the present invention takes α-helix structure in the presence of a particular substance. For example, the peptide takes α-helix structure through interaction with an amphipathic substance such as a surfactant or a particular phospholipid on the cell membrane such as phosphatidyl serine, and this invites increase in the permeability of the peptide or the substance containing the peptide through cell membrane, and smooth migration of the peptide or the substance containing the peptide into the cell.

A preferred embodiment of the peptide according to the first aspect of the present invention is a peptide which does not exhibit α-helix structure in the CD spectroscopy in an aqueous solution containing no solute or in an aqueous solution containing only an inorganic salt at a pH of 5 to 8, but which shows two local minimums in the mean residue ellipticity in the CD spectroscopy in an aqueous solution containing 5mM SDS at a pH of 5 to 8 at two wavelength regions, namely, at the wavelength region of 205 to 210 nm and at the wavelength region of 220 to 225 nm, indicating the α-helix structure.

The peptide is preferably the one wherein the proportion of the α-helix structure in the higher order structure is 25% or more when calculated by the method of Chen et al. More preferably, the peptide is the one wherein the proportion of the α-helix structure is 30% or more, and still more preferably, the one wherein the proportion of the α-helix structure is 35% or more.

Such peptide is found to take an α-helix structure in an aqueous solution in the presence of an amphipathic substance. In addition, since such peptide has affinity for phosphatidyl serine, such peptide selectively accumulates on the surface of an abnormal cell, for example, on the surface of an injured, denatured, or activated cell, and takes the α-helix structure through interaction with a phosphatidyl serine on the cell membrane, and the peptide is then selectively incorporated in the cell.

The peptide according to the first aspect of the present invention is preferably the one which does not form aggregates in the presence of a protein.

For example, in the case of the peptide according to the preferred embodiment of the present invention, the peptide will not take an α-helix structure in the absence of an amphipathic substance, and solubility will be maintained in the presence of proteins. As a consequence, the peptide is prevented from forming aggregates that may cause substantial problem when administered to a human, and the risk of the blood vessel occlusion is reduced with an increased safety. Example 26, for example, demonstrates the high safety of the peptide according to the first aspect of the present invention.

Whether or not the peptide according to the first aspect of the present invention forms the aggregates to a substantial level in the presence of a protein can be determined by optically measuring turbidity of an aqueous solution of serum albumin containing the peptide, for example, by measuring absorption of the aqueous solution of serum albumin containing the peptide at a wavelength of 340 nm to 660 nm, and in particular, at a wavelength of 600 nm.

The peptide of the present invention is useful as a peptide vector because of its characteristic features that the peptide easily binds to the nucleic acid, that it exhibits high solubility after forming the complex with the nucleic acid, that it is highly capable of introducing the nucleic acid in the cell, that it demonstrates the high safety, and that it has affinity for phosphatidyl serine in the presence of serum albumin enabling its selective incorporation into the abnormal cell.

A peptide vector is a peptide which is capable of introducing the desired substance into a cell, its derivative, its modification, or its analog.

The peptide of the present invention also has a characteristic feature that it can prevent decomposition of the nucleic acid by a nuclease. Accordingly, a natural type oligonucleotide or polynucleotide (P=O form) which is easily decomposed by the nuclease in the blood or cell becomes resistant to the decomposition by the nuclease once the peptide of the present invention is bonded to such oligonucleotide or polynucleotide.

Whether or not the peptide imparts the nuclease resistance to the nucleic acid can be determined by allowing the complex of the nucleic acid and the peptide of the present invention to react with the nuclease in a solution containing the nuclease, extracting the nucleic acid, and subjecting the extracted nucleic acid to electrophoresis and detecting the stained image. For example, if the peptide imparts the nucleic acid with the nuclease resistance, the nucleic acid will remain intact and the stained image of the nucleic acid will be obtained. Examples 18 and 19 recite the detailed procedure.

When the peptide of the present invention is used as a peptide vector, a substance capable of binding to the vector, which is preferably a nucleic acid, can be introduced into the cell, and development of the function of the nucleic acid in the cell is realized by such introduction in the cell of the nucleic acid. Examples 11 to 16 are directed to the embodiments wherein the peptide of the present invention is used to an in vitro introduction of a nucleic acid into the cell, and the firefly luciferase and HSV-tk coded by the nucleic acid are respectively expressed in the cell. Examples 24 and 25 are directed to the embodiments wherein the peptide of the present invention is used to an in vivo introduction of a nucleic acid in the cell, and the firefly luciferase and HSV-tk coded by the nucleic acid are respectively expressed in the cell. In particular, Example 25 is directed to the embodiment wherein the HSV-tk gene that has been introduced in the tumor cell by using the peptide of the present invention is expressed in the tumor cell, and ganciclovir sensitivity of the tumor cell is thereby enhanced to exhibit the pharmacological action (anti-tumor action). The dose of the plasmid used in this Example was 10 µg, and this dose was by far smaller than the dose (150 µg) used in similar pharmacological experiment using the conventional liposome vector (Aoki, K. et al., Hum. Gene Ther., Vol. 8, 1105 (1997)). As will be more illustratively shown in Examples 17 and 18, the peptide of the present invention increases the stability of the nucleic acid to an extent further than the liposome, and the peptide of the present invention that has become bonded to the nucleic acid has a stability higher than that of the liposome, and therefore, use of the peptide of the present invention in introducing a nucleic acid in a cell is useful, and the peptide of the present invention is excellent for use as a vector in introducing a nucleic acid in a cell.

Use of the peptide of the present invention as a vector other than the embodiments as described above include use of the peptide in introducing the following substances into the cell to thereby allow development in the cell of the function of the following substances: a plasmid expressing a reporter protein (green fluorescent protein (GFP), β-galactosidase, etc.); a plasmid expressing a cytokine (interleukin 2, interferon β, etc.) which exhibits anti-tumor effects; a plasmid expressing a physiologically active substance (Fas ligand, p53, caspase 3, caspase 8, Bax (Bcl-2-associated X protein), FADD (Fas associated death domain protein), etc.) which induces apoptosis to exert cytotoxic effects; a plasmid expressing a soluble receptor for a ligand such as TNF-α or interleukin 6, which competitively binds to the ligand to suppress the reaction induced by the ligand, and which can thereby improve the symptom of, for example, chronic articular rheumatism; a plasmid expressing a peptide/polypeptide which can serve a vaccine to suppress an allergic reaction or a protein such as mite antigen which is an antigenic protein; a plasmid expressing vascular endothelial growth factor (VEGF) or hepatocyte growth factor (HGF) which has the action of improving the pathological condition of arteriosclerosis obliterans (a circulatory disease) or healing (remodeling) the injured lesion; an antisense oligonucleotide or a ribozyme for CDC2 kinase which has the action of suppressing the restenosis after percutaneous transluminal coronary angioplasty (PTCA); a decoy for a nucleotide sequence of to which E2F (a transcriptional regulatory factor for a cell cycle regulatory gene) or NFκB (a transcriptional regulatory factor for a cytokine) binds; a phosphorylated nucleic acid analog in its active form which exhibits an anti-virus action; and the like.

Since the peptide of the present invention has the ability of binding to a nucleic acid, ability of introducing the nucleic acid to a cell, and affinity for phosphatidyl serine in the presence of serum albumin as its characteristic features, the peptide will introduce the nucleic acid at a higher efficiency to a cell wherein a larger amount of phosphatidyl serine has been translocated to the surface. This means that, when a nucleic acid such as a gene or an antisense DNA is to be introduced in a cell for the purpose of treating a disease, the nucleic acid will be selectively introduced in a larger amount to an abnormal cell such as an injured, denatured, or activated inflammatory cell, or to an immunocompetent cell wherein an increased amount of phosphatidyl serine has been translocated to the cell surface. Reduction of the side effects is thereby attained.

For example, when the peptide of the present invention is used as a vector in introducing a nucleic acid to a tumor cell for the purpose of treating a cancer, the nucleic acid is scarcely introduced to a normal cell, while the nucleic acid is introduced to the tumor cell at a high rate. When a nucleic acid is introduced in a cell for the purpose of treating an allergy, the nucleic acid is specifically introduced to a cell wherein allergic reaction has been induced by the immunocompetent cell.

Furthermore, the nucleic acid can be introduced at a higher rate to the desired particular cell or organ by utilizing the capability of the peptide of the present invention "to introduce the nucleic acid at a higher rate to the cell wherein a larger amount of phosphatidyl serine has been translocated to the cell surface", and namely, by preliminarily increasing the amount of the phosphatidyl serine translocated to the surface of the desired particular cell or organ. To be more specific, introduction of the nucleic acid at a higher rate to the desired particular cell or organ can be realized by reacting a reagent with the particular cell or organ to thereby increase the amount of the phosphatidyl serine translocated to the surface of the particular cell or organ by the pharmacological action of the reagent, and thereafter using the peptide of the present invention as a vector. For example, a therapy is still possible even if a chemotherapeutic treatment of a tumor by sole administration of an anticancer drug failed to achieve sufficient therapeutic effects, and in such a case, the chemotherapeutic agent may be administered to increase they amount of the phosphatidyl serine translocated to the surface of the tumor cell by the pharmacological action of the chemotherapeutic agent, and then, the peptide of the present invention may be used as a vector to thereby realize the highly efficient introduction of a gene, the antisense DNA, or other nucleic acid which exhibits high anti-tumor effects into the tumor cell and utilize the improved therapeutic effects of the nucleic acid.

As described above, the peptide of the present invention readily binds to a nucleic acid and introduces the nucleic acid into the cell. The peptide of the present invention, however, is not only capable of introducing a nucleic acid, but also capable of introducing any substance which binds to the peptide of the present invention. As in the case of the binding of the peptide of the present invention with the nucleic acid wherein the mode of the binding is not particularly limited, the mode of the binding is not limited in the case of the binding of the peptide of the present invention with such substance. The mode of the binding between the peptide of the present invention and such substance upon introduction of such substance into the cell is preferably a noncovalent bond, more preferably an electrostatic bond, and most preferably an electrostatic bond established between the positive charge of the peptide of the present invention and the negative charge of the substance. In other words, the present invention includes within its scope an embodiment wherein a substance which binds to the peptide of the present invention by the mode of the binding as described above is introduced into a cell. Exemplary substances which binds to the peptide of the present invention and which are other than the nucleic acids include low molecular weight compounds having an acidic protein or ammonium group as their side chain.

Furthermore, the peptide of the present invention is not particularly limited for its peptide length, amino acid sequence, addition of sugar chain, modification, and the like as long as features of the peptide of the present invention are maintained, and any peptide having the features of the peptide of the present invention is within the scope of the peptide according to the first aspect of the present invention. For example, the peptide according to the first aspect of the present invention may include addition of a sugar chain or a lipid or modification by a high molecular weight compound for the purpose of improving the in vivo stability, and/or addition of a sugar chain or a lipid or modification by a high molecular weight compound for the purpose of suppressing the recognition of the peptide by the antigen presenting cell. To be more specific, the peptide may be modified with mannose, cholesterol, or polyethylene glycol.

The peptide of the present invention can be produced by chemical synthesis, for example, by a synthesis using an automatic peptide synthesizer (432A, manufactured by Applied Biosystems).

The peptide of the present invention may also be produced by a genetic engineering means. When the peptide is produced by genetic engineering methods, the desired peptide may be produced by the following steps:
(1) the step of producing a DNA having the nucleotide sequence coding for the amino acid sequence of the peptide;
(2) the step of introducing the DNA in a vector to thereby produce an amplifiable recombinant DNA including the DNA;
(3) the step of transforming a host cell with the recombinant DNA to produce a transformant capable of expressing the peptide; and
(4) the step of cultivating the transformant to produce the peptide, and recovering the peptide from the culture mixture.

The DNA coding for the peptide of the present invention may be any DNA having the nucleotide sequence which substantially codes for the peptide of the present invention. As is well known in the art, because of the degeneration of codon, at least one nucleotide in the gene sequence can be replaced with another nucleotide without causing any change in the amino acid sequence of the peptide coded by the gene sequence. Therefore, the DNA may have a nucleotide sequence wherein at least one nucleotide in the nucleotide sequence has been replaced on the bases of the degeneration of the genetic code. To be more specific, when the peptide of the present invention is produced by using genetic engineering methods, the peptide may have a nucleotide sequence wherein at least one nucleotide has been replaced so that the codon will be the one frequently found in a particular host cell. In addition, the DNA may be a recombinant DNA, such as a plasmid or an expression vector.

Exemplary DNAs of SEQ ID NO:28 to SEQ ID NO:30 coding for the peptides of SEQ ID NO:1, SEQ ID NO:16, and SEQ ID NO:19 are shown.

The step of obtaining the DNA having a nucleotide sequence coding for the amino acid sequence of the peptide may be accomplished by the synthesis using an automatic nucleic acid synthesizer.

The step of incorporating the DNA in the vector to obtain an amplifiable recombinant DNA including the DNA, and the step of transforming the host cell by the recombinant DNA to obtain a transformant which is capable of expressing the peptide may be accomplished by the genetic engineering methods generally used in the art as described in a book (for example, Molecular Cloning: a laboratory manual, Second edition, T. Maniatis et al., Cold Spring Harbor Laboratory Press (1989)). It is to be noted that, since the peptide of the present invention can be designed as a peptide including no methionine, the peptide can be obtained by producing a peptide wherein a plurality of the peptide according to the first aspect of the present invention are ligated by the intervening methionine by genetic engineering methods, and thereafter cleaving the peptide with cyanogen bromide.

The peptide produced may be purified, isolated, and recovered by referring to methods described in various articles and books (for example, "Experiments in Biochemistry: A New Lecture Series: Protein I" (Japanese Society of Biochemistry, ed., Tokyo Kagaku-Dojin, 1990), "Kagaku, Special edition, 102: high performance liquid chromatography of proteins and peptides" (N. Ui et al., ed., Kagaku-Dojin, 1985)). To be more specific, the peptide produced may be obtained in its pure form by using at least one of the procedures selected from demineralization, concentration, salting out, ultrafiltration, ion exchange chromatography, reversed phase chromatography, isoelectric chromatography, affinity chromatography, and gel permeation.

As will be described below, the peptide of the present invention may be used as a peptide which constitutes the vector according to the second aspect of the present invention. As will also be described below, the peptide of the present invention may also be used as a carrier in the delivery method according to the fifth aspect of the present invention.

The peptide of the present invention may also be used as a research tool, for example, as a tool in gene cloning. To be more specific, microorganisms and cells can be transformed at an improved efficiency by using the peptide of the present invention, and therefore, use of the peptide according to the present invention enables cloning at an improved efficiency of the gene coding for a physiologically active substance, for example, an enzyme or its inhibitor, a receptor involved in signal transduction, a ligand or its associated factor, a factor involved in immune mechanism, a factor involved in hematopoiesis or blood coagulation, a factor involved in cell differentiation or propagation, and a factor involved in gene transcription or translation.

The gene cloning using the peptide of the present invention may be accomplished by the following steps:
(1) the step of extracting mRNA by lysing cells or tissue;
(2) the step of synthesizing cDNA by using the mRNA for the template;
(3) the step of introducing the synthesized cDNA to a vector such as a plasmid or a phage;
(4) the step of forming a complex of the vector having the cDNA introduced therein and the peptide of the present invention;
(5) the step of transforming a microorganism or a cell by using the complex;
(6) the step of cultivating the transformed microorganism or cell, and selecting the desired gene by using a probe or an assay system; and
(7) the step of determining nucleotide sequence of the selected gene.

Another example of the use of the peptide of the present invention for the research tool is its use for the screening carried out by genetic engineering methods. To be more specific, a cell can be transformed at an improved efficiency by using the peptide of the present invention, and therefore, a substance which interacts with a physiologically active substance or a substance involved in the expression of the physiologically active substance can be screened at an improved efficiency. Exemplary physiologically active substances include an enzyme or its inhibitor, a receptor involved in signal transduction, a ligand or its associated factor, a factor involved in immune mechanism, a factor involved in hematopoiesis or blood coagulation, and a factor involved in cell differentiation or propagation. Exemplary substances involved in the expression of the physiologically active substance include a factor involved in gene transcription or translation.

The screening using the peptide of the present invention may be accomplished by the following steps:
(1) the step of obtaining the DNA coding for a physiologically active substance or a reporter protein;
(2) the step of constructing an expression vector by adding a transcriptional regulatory region (promoter, enhancer, etc.) to the DNA;
(3) the step of forming a complex of the expression vector and the peptide of the present invention;
(4) the step of transforming a cell with the complex;
(5) the step of cultivating the transformed cell in the presence of the substance to be screened; and
(6) the step of quantitating the physiologically active substance or the reporter protein that had been expressed.

Another example of the use of the peptide of the present invention for the screening carried out by genetic engineering methods is use of the peptide in the screening of antisense oligonucleotide. Since the peptide of the present invention imparts natural type (P=O form) oligonucleotide with nuclease resistance as described in Example 18, an effective antisense sequence can be detected without being influenced by the toxicity when the P=O form oligonucleotide with no toxicity is reacted with the cell together with the peptide of the present invention in the presence of serum.

The screening of the effective antisense sequence using the peptide of the present invention may be accomplished by the following steps:
(1) the step of constructing a vector which expresses the physiologically active substance that can serve the target;
(2) the step of synthesizing an antisense oligonucleotide against the expression vector;
(3) the step of forming a complex of the antisense oligonucleotide and the peptide of the present invention;
(4) the step of cultivating a cell which has been transformed by the vector which expresses the physiologically active substance serving as the target, in the presence of the complex: and
(5) the step of quantitating the physiologically active substance that had been expressed.

A further example of the use of the peptide of the present invention for the research tool is its use in the production of an antibody. To be more specific, when a plasmid expressing an antigen which serves the target for the desired antibody is administered with the peptide of the present invention to muscle, spleen, peritoneal cavity, or the like of an animal, and preferably, a mouse, the desired antibody can be produced in a convenient manner without preparing the purified antigen.

The production of the antibody using the peptide of the present invention may be accomplished by the following steps:
(1) the step of constructing a plasmid which expresses the antigen;
(2) the step of forming a complex of the plasmid and the peptide of the present invention;
(3) the step of administering the complex at least twice at a predetermined time interval to muscle, spleen, or abdominal cavity of an animal, and preferably, of a mouse; and
(4) the step of collecting blood and purifying the antibody.

The peptide of the present invention can also be used as a diagnostic agent. In such a case, the peptide of the invention may be used, for example, after labeling the peptide with a label compound such as a radioisotope. Administration of the labeled peptide enables identification of the sites such as the site where inflammation or cell activation and/or injury, apoptosis, or other immune responsive reaction caused by the immunocompetent cell has taken place, the site where cells have become malignantly transformed through the progress of abnormal cell division, the site where the cells constituting the blood vessel have been injured by blood coagulation or by the progress of arterial sclerosis, the site where cytotoxic reaction induced by an active enzyme is in progress, or the site where cell activation and/or cell injury induced by a protease is in progress. Such sites include the so called abnormal cells which had been injured, denatured, or activated, and the peptide of the present invention, which has specific affinity for phosphatidyl serine which is a phospholipid whose content in the outer layer of the lipid bilayer increases in such abnormal cell, will bind to such site or such cell, and the diagnosis of the lesion by radiography is thereby enabled. The peptide of the present invention may also be used in diagnosing the conditions of the lesion after administering an anticancer drug or an immunosuppressant, namely, in diagnosing the presence or absence of apoptosis.

The embodiments as described above are only few of the utilization of the peptide of the present invention in the research wherein the substance which binds to the peptide of the present invention is introduced in the interior of a cell, and the utilization of the peptide of the present invention is not limited to those described above.

Second aspect of the present invention is directed to a vector for gene therapy containing the peptide according to the first aspect of the present invention. The gene therapeutic vector of the present invention is not limited as long as it contains the peptide of the present invention. The type of the gene therapy is also not limited. The peptide of the present invention has been described in the section of the peptide of the present invention.

The vector of the present invention is not particularly limited for how the vector and the gene are present. The vector may be bonded to the gene by electrostatic bond, hydrophobic bond, or other noncovalent bond; disulfide bond, ester linkage, ether linkage, peptide bond, or other covalent bond; or a mixture of such binding modes. Alternatively, the vector and the gene may be simply a mixture of the both, or the vector and the gene may form a composition.

The blend ratio of the vector of the present invention and the gene is not particularly limited as long as the genetic substance becomes efficiently bonded to the peptide of the present invention to form a complex that is efficiently introduced in the cell. The ratio, however, is preferably such that the ratio (+/- ratio) of the number (+) of groups having positive charge of the peptide of the present invention to the number (-) of the groups having negative charge of the nucleic acids is 2 or more, and more preferably 3 or more. The ratio, on the other hand, is preferably up to 100, and more preferably up to 50.

When the vector of the present invention is used for gene therapy, the composition of the vector may be a mixture with a pharmacologically effective additive as long as the function of the vector and the gene are maintained. Exemplary such additives include sterilized water, physiological saline, vegetable oil, mineral oil, higher alcohol, higher fatty acid, nontoxic organic solvent, or other pharmacomedical carrier or medium, and the composition may further include excipient, colorant, emulsifier, suspending agent, surfactant, solubilizer, adsorption-preventing agent, stabilizer, preservative, humectant, antioxidant, buffer agent, isotonic agent, pain-reducing agent, or the like as desired. Use of a solvent such as polyethylene glycol, or glucose or other sugar solution is particularly preferable in view of further increasing the solubility. After adding such additives, the vector of the present invention may be formulated in the form of a pharmaceutical composition or a kit such as an injectable preparation, oral preparation, instillation, aerosol, or external medicine.

The vector of the present invention may be administered either orally or parenterally, and preferably, parenterally, for example, by intravenous injection, intracoronary injection, intramuscular injection, intraperitoneal injection, or subcutaneous injection; instillation; or tracheobronchial spray or intranasal spray; and either systemically or locally, and either rapidly or continuously. Use of the vector of the present invention, however, is not limited to such administration method, and also, the vector of the present invention may be used with other reagents.

The dose of the gene used in combination with the vector of the present invention depends on the physiological activity of the gene, and the dose is not particularly limited as long as an effective dose for in vivo action of the gene is administered. The dose may be determined depending on the conditions of the patients and the like. The dose, however, is preferably 0.1% to 10% of the effective dose of the substance when the gene is administered alone.

As specifically described in the section of the peptide of the present invention by referring to the Examples, the content of the gene is significantly lower than the content of the gene when the gene is used as a mixture with dioctadecylamidoglycylspermine (hereinafter referred to as DOGS) or other cationic liposome. For example, the content is 1/2 or less, preferably, 1/5 or less, more preferably 1/10 or less, and most preferably 1/20 or less.

To be more specific, the dose of the gene used in combination with the vector of the present invention is preferably 10 ng/kg to 10 mg/kg, and the dose of the peptide of the present invention constituting the vector of the present invention is preferably 20 ng/kg to 1 g/kg.

The vector of the present invention has the characteristic features of the peptide of the present invention, and it exhibits high solubility, high capability of being introduced into a cell, high safety, and high affinity for phosphatidyl serine in the presence of serum albumin.

The vector of the present invention and the gene used in combination with the vector of the present invention have a characteristic feature of high resistance to decomposition by nuclease and the like, and therefore, higher order structure does not change with lapse of time. As a consequence, efficiency in the introduction into the cell and affinity for phosphatidyl serine are not degraded with lapse of time, and storage stability is excellent. The storage stability may be evaluated by storing at 4°C for 1 week, and measuring the amount of the gene expressed. The procedure of such evaluation will be described in detail in Example 17.

Since the phosphatidyl serine has the characteristics as described above, the vector of the present invention is selectively accumulated on the surface of the abnormal cell such as the cell in the inflammatory lesion that has been injured, denatured, or activated, and becomes incorporated in the cell together with the gene. This is quite favorable aspect of the vector of the present invention in view of reducing the side effects when the gene is administered in combination with the vector of the present invention for the purpose of treating a disease.

For example, when the vector of the present invention is used in treating cancer, the gene used in combination with the vector is little introduced in the normal cell while the gene is conveniently introduced in the tumor cell at a high efficiency. Exemplary such combinations include the vector of the present invention used in combination' with a gene coding for a protein such as p53 exhibiting anticancer action, and the vector of the present invention used in combination with a gene coding for a protein which activates the anticancer drug optionally in further combination with an anticancer drug. Such use is described, for example, in Example 15. Another example is the case of introducing a gene in a cell for the purpose of treating allergy, and use of the gene in combination with the vector of the present invention is favorable in such a case since the gene will be specifically introduced in the cell undergoing the allergic reaction. Such use is described, for example, in Example 23.

Since the vector of the present invention exhibits specific affinity for phosphatidyl serine, use of the vector of the present invention with other drug is also favorable. In such a case, the vector of the present invention will be highly incorporated to the cell or organ wherein the amount of the phosphatidyl serine translocated to the surface has been increased by the action of the drug. For example, in the case of chemotherapeutic treatment of a cancer, therapeutic effects can be realized by the use of the vector of the present invention even if sufficient therapeutic effects were not realized by solely administering an anticancer drug, since the vector of the present invention is introduced at an improved efficiency to the tumor cell wherein the amount of the phosphatidyl serine has been increased by the action of the anticancer drug. Alleviation of the side effects is also realized since the dose of the anticancer drug can be reduced.

An example of the gene used in combination with the vector of the present invention is a plasmid. To name a few of such plasmids which can be used as an antitumor drug (anticancer drug) or an antiinflammatory drug, there are a plasmid expressing a cytokine (interleukin 2, interferon β, etc.) which exhibits anti-tumor effects; a plasmid expressing a physiologically active substance (Fas ligand, p53, caspase 3, caspase 8, Bax (Bcl-2-associated X protein), FADD (Fas associated death domain protein) etc.) which induces apoptosis to develop cytotoxic effects; a plasmid expressing HSV-tk which enhances ganciclovir sensitivity of the tumor cell and develops cytotoxic effects; a plasmid expressing a soluble receptor for a ligand, which is capable of improving the inflammatory pathological conditions such as chronic articular rheumatism by competitively binding to the ligand such as TNF-α or interleukin 6 and suppressing the reaction induced by such ligand; and a plasmid expressing a protein which is a peptide/polypeptide which serves a vaccine to suppress an allergic reaction or which is an antigen protein such as mite antigen. Exemplary plasmid which can be used as a drug for arteriosclerosis obliterans which is a circulatory disease is a plasmid expressing vascular endothelial growth factor (VEGF) or hepatocyte growth factor (HGF).

Other examples of the gene which may be used in combination with the vector of the present invention are antisense oligonucleotides, ribozymes, and decoys. An antisense oligonucleotide or a ribozyme for CDC2 kinase which is a cell cycle regulatory gene, and a decoy for the nucleotide sequence to which E2F which is a transcriptional regulatory factor for a cell cycle regulatory gene or NFκB which is a transcriptional regulatory factor for an inflammatory cytokine bound to the vector of the present invention are examples of the gene which may be used for restenosis after percutaneous transluminal coronary angioplasty (PTCA) which is a circulatory disease. An antisense oligonucleotide for herpes simplex virus is an example for an antiviral agent. The vector of the present invention may also be used in combination with a phosphorylated nucleic acid analog in activated form. This gene is an exemplary antiviral agent.

Table 1 shows examples of the genes including those described above whose combination with the vector of the present invention having specific affinity for a phospholipid is particularly effective. The intended diseases are also shown in Table 1.

The vector of the present invention can be used not only for the applications wherein the gene is administered directly to the interior of the body but also for ex vivo applications wherein the gene is introduced in the cell that has been taken out of the body, the introduction procedure being carried out outside the body, and then, the cell is returned to the body. An exemplary such ex vivo use is use of the vector of the present invention with GM-CSF gene. When the GM-CSF gene is administered in vitro to a tumor cell that has been taken out of a cancer patient body, the GM-CSF gene is introduced into the tumor cell by the vector of the present invention, and the GM-CSF is expressed in the cell. When this cell is returned to the body of the cancer patient, ability of the antigen presenting cell to present the tumor antigen is enhanced, and the anti-tumor immunological effects of the cytotoxic immunocompetent cell are increased. The anti-tumor effects are thereby developed.

Third aspect of the present invention is directed to a composition for gene therapy containing the peptide of the present invention and a plasmid including herpes simplex virus thymidine kinase (HSV-tk) gene. The gene therapeutic composition of the present invention contains the peptide of the present invention, a plasmid having herpes simplex virus thymidine kinase (HSV-tk) gene incorporated therein, and optionally, one or more pharmacologically acceptable additives. The gene therapeutic composition of the present invention may be produced as an injectable preparation, oral preparation, instillation, aerosol, or external medicine by adding and mixing an excipient, colorant, emulsifier, suspending agent, surfactant, solubilizer, adsorption-preventing agent, stabilizer, preservative, humectant, antioxidant, buffer agent, isotonic agent, pain-reducing agent, or the like as desired.

HSV-tk phosphorylates ganciclovir or acyclovir which is an antiviral agent, and the phosphorylated product is further phosphorylated by cell's endogenous thymidine kinase, and the thus tri-phosphorylated ganciclovir or acyclovir inhibits DNA synthesis of the cell, thus suppressing the cell propagation. Accordingly, introduction of the HSV-tk gene together with the administration of the ganciclovir or the acyclovir is effective in the case of the disease which is caused by cell propagation.

In other words, when the composition according to the third aspect of the present invention containing the peptide of the present invention and the plasmid having HSV-tk gene incorporated therein is produced as a composition further containing ganciclovir or acyclovir, the composition is effective in treating cancer, arterial sclerosis, and the like and in preventing the restenosis after percutaneous transluminal coronary angioplasty, and GVHD.

Fourth aspect of the present invention is directed to a method for treating or preventing cancer, tumor, arterial sclerosis, restenosis after percutaneous transluminal coronary angioplasty, or GVHD which is characterized by administering a gene therapeutic composition containing the peptide of the present invention and a plasmid having herpes simplex virus thymidine kinase (HSV-tk) gene incorporated therein, administering ganciclovir or acyclovir simultaneously with or after the administration of the gene therapeutic composition, and administering ganciclovir or acyclovir for at least for another 5 days.

The method for treating or preventing cancer, tumor, arterial sclerosis, restenosis after percutaneous transluminal coronary angioplasty, or GVHD of the present invention is characterized by administering a gene i therapeutic composition containing the peptide of the present invention and a plasmid having herpes simplex virus thymidine kinase (HSV-tk) gene incorporated therein, administering ganciclovir or acyclovir simultaneously with or after the administration of the gene therapeutic composition, and administering ganciclovir or acyclovir for at least for another 5 days, and preferably for one week. More preferably, the administration of the ganciclovir or the acyclovir is continued for the period while expression of the HSV-tk can be expected. Such period which may vary from individual to individual is generally in the range of 7 days to 10 days. Most preferably, if the expectable period for the HSV-tk expression had passed, the process as described above is restarted from the administration of the gene therapeutic composition to continue the administration of the ganciclovir or the acyclovir.

The dose which may vary from individual to individual is preferably 1 mg/kg to 100 mg/kg in the case of ganciclovir. The dose of the plasmid having HSV-tk gene incorporated therein which may vary by the size of the plasmid is preferably 10 ng/kg to 10 mg/kg, and the dose of the peptide of the present invention used with the plasmid of such dose is 20 ng/kg to 1g/kg.

Fifth aspect of the present invention is directed to a method for delivering a substance which binds to the peptide of the present invention by using the peptide as a carrier.

The substance which binds to the peptide of the present invention used in the method according to the fifth aspect of the present invention is not particularly limited as long as the substance is capable of binding to the peptide, and the binding of the substance with the peptide does not completely disturb the affinity of the peptide for phosphatidyl serine or the function of the substance itself. Furthermore, the mode of the binding between such substance and the peptide is not limited, and the substance may be any substance which binds to the peptide by an electrostatic bond, hydrophobic bond, or other noncovalent bond; disulfide bond, ester linkage, ether linkage, peptide bond, or other covalent bond; or a mixture of such binding modes. Among these, the preferred are those which bind to the peptide by a noncovalent bond, and the more preferred are those which bind to the peptide by an electrostatic bond. The most preferred are those wherein the bonding is established by the electrostatic bond between the positive charge of the peptide and the negative charge of the substance, and an exemplary such substance is nucleic acid.

The substance which binds to the peptide is expected to exhibit its action when it is delivered into the interior of the cell by the method according to the fifth aspect of the present invention. An exemplary such substance which binds to the peptide and exhibits its action is a physiologically active substance, and the substance is more preferably a physiologically active substance whose physiological activity is not totally lost when it is administered to a human or an animal by binding to the peptide serving the carrier, and which is introduced into the interior of the cell substantially with the peptide. Exemplary such physiologically active substances include nucleic acids, physiologically active peptides, lipids, and low molecular weight compounds, and the substance is more preferably a nucleic acid which may be an antisense oligonucleotide, a nucleic acid which contains a sequence useful in the gene therapy, a derivative thereof, a modification thereof, an analog thereof, a mixture thereof, or an inclusion body thereof.

The delivery method according to the fifth aspect of the present invention may be either an in vitro method or an in vivo method.

In the case of an in vitro method, the peptide of the present invention may be mixed with or bonded to the substance which binds to the peptide, and the thus produced complex may be added to a cell culture to continue the cultivation.

In the case of an in vivo method, the peptide of the present invention may be mixed with or bonded to the substance which binds to the peptide, and after optionally adding pharmacological effective additives, the mixture may be administered to a human or an animal. In the in vivo method, administration may be accomplished either systemically or locally, and either rapidly or continuously, by parenteral administration such as intravenous injection, intracoronary injection, intramuscular injection, intraperitoneal injection, or subcutaneous injection; instillation; or tracheobronchial spray or intranasal spray.

In both the in vitro and the in vivo methods, the peptide of the present invention forms a complex with the substance which binds to the peptide to prevent the decomposition of the substance, and this complex accumulates selectively on the surface layer of the cell, and preferably on the surface of an abnormal cell, for example, on the surface of an injured, denatured, or activated cell. The complex is thereby incorporated in the cell at a high efficiency, and the substance which binds to the peptide is thereby delivered into the interior of the cell. Accordingly, the substance is introduced at a higher rate in the abnormal cell in which phosphatidyl serine has been translocated to the cell surface. As described above, the method according to the fifth aspect of the present invention is a method which is quite favorable in view of reducing the side effects when the substance is to be introduced in the cell for the purpose of disease treatment. For example, when a tumor is to be treated by introducing the substance to the tumor cell, use of the method according to the fifth aspect of the present invention is convenient since the substance is little introduced in the normal cell while the substance is introduced in the tumor cell at a high rate as shown Example 15 thereby exhibiting the anti-tumor action. When the substance is introduced in the cell for the purpose of treating allergy, use of the method according to the fifth aspect of the present invention is convenient since the substance is introduced in the cell undergoing an allergic reaction in a specific manner as shown in Example 23.

Examples 10, 11, 15, 16, and 22 to 25 are illustrative examples of the method according to the fifth aspect of the present invention wherein the "peptide which has ability to bind to the nucleic acid, ability to introduce the nucleic acid to the cell, and affinity for the desired phospholipid" has been used for the carrier. The complex formed by the binding of such peptide and the nucleic acid was confirmed to exhibit an increased accumulation to particular phospholipid and a significantly improved introduction into the interior of the cell.

By using the substance which has affinity for phosphatidyl serine in the presence of serum albumin for the carrier as described below, the delivery method according to the fifth aspect of the present invention provides a method for delivering a physiologically active substance, which is preferably a nucleic acid, to the interior of the cells at the site such as the site where inflammation or cell activation and/or injury, apoptosis, or other immune responsive reaction such as allergy caused by the immunocompetent cell has taken place, the site where cells have become malignantly transformed through the progress of abnormal cell division, the site where the cells constituting the blood vessel have been injured or propagated by blood coagulation or by the progress of arterial sclerosis, the site where cytotoxic reaction induced by an active enzyme is in progress, or the site where cell activation and/or cell injury induced by a protease is in progress. It is to be noted, however, the method of the present invention is not limited to the embodiments as described below.

As will be described in Examples 16 and 25, exemplary such methods include a method wherein a plasmid expressing HSV-tk which enhances ganciclovir sensitivity of the tumor cell and develops cytotoxic effects is introduced in the tumor cell by using a substance which exhibits affinity for phosphatidyl serine in the presence of serum albumin for the carrier, and a method wherein a plasmid expressing a physiologically active substance (Fas ligand, p53, caspase 3, caspase 8, Bax (Bcl-2-associated X protein), FADD (Fas associated death domain protein) etc.) which exhibits cytotoxic effects on propagated synovial cell in the lesion of rheumatoid arthritis, infiltrated lymphocyte, or cancer cell is introduced in the cell by using a substance which exhibits affinity for phosphatidyl serine in the presence of serum albumin for the carrier.

Another embodiment is a method wherein a physiologically active substance, which is preferably a nucleic acid, is delivered to the interior of an immunocompetent cell (for example, mast cell or basophil) in the tissue where an allergic reaction is in progress, by using a substance which exhibits affinity for phosphatidyl serine in the presence of serum albumin for the carrier. In the tissue of the skin of an atopic disease patient or the site of asthma seizure, for example, lung or trachea of the patient, mast cells and basophils are undergoing degranulation which has been caused by the binding of the allergen to the IgE antibody on the cell surface, and in such a cell, phosphatidyl serine that had been contained in the granule has been translocated to the surface of the cell in the course of the degranulation. Therefore, the delivery method according to the fifth aspect of the present invention is particularly useful as a method for delivering the physiologically active substance, which is preferably a nucleic acid, to the interior of such cell as demonstrated in Example 23.

Another embodiment is a method wherein a plasmid expressing VEGF or HGF which has an remodelling action for the cells constituting the blood vessel which has been injured by blood coagulation or by the progress of arterial sclerosis, the cells in the site where cytotoxic reaction caused by super oxide is in progress, or the cells in the site wherein cell activation and/or cell injury caused by a protease is in progress is delivered to the interior of the cell by using a substance which exhibits affinity for phosphatidyl serine in the presence of serum albumin for the carrier.

A further embodiment is a method wherein a physiologically active substance, preferably a nucleic acid, and more preferably a nucleic acid analog in activated-form which is used as an antiviral agent or an anticancer drug is delivered into the interior of the cell constituting the tissue or organ of the virus-infected site or the like by using a substance which exhibits affinity for phosphatidyl serine in the presence of serum albumin for the carrier. Although such nucleic acid analog in activated-form has been phosphorylated and the amount of such phosphorylated nucleic acid incorporated into the cell is generally reduced, efficient incorporation of the phosphorylated nucleic acid analog into the cell is enabled by the binding to the carrier preferably by an electrostatic bond. In other words, the delivery method of the present invention has enabled the incorporation of the nucleic acid analog into the cell of the particular desired tissue in its activated, phosphorylated state.

A still further embodiment is a method wherein a physiologically active substance, preferably a nucleic acid is delivered into the interior of the cell constituting brain tissue by using a substance which exhibits affinity for phosphatidyl serine in the presence of serum albumin for the carrier. An embodiment of such method is introduction of interferon β-expressing plasmid for the purpose of treating brain tumor. Since the cell constituting the brain tissue has high content of phosphatidyl serine, the delivery method according to the fifth aspect of the present invention is particularly useful as a method for delivering a physiologically active substance, which is preferably a nucleic acid, to the interior or the cell constituting the brain tissue.

As described above, the delivery method according to the fifth aspect of the present invention is capable of site-specifically introducing the physiologically active substance, and preferably a nucleic acid, into the interior of the cell, and this has enabled a remarkable enhancement of the action of the physiologically active substance.

In other words, incorporation of the physiologically active substance, preferably a nucleic acid, in the cell at an improved efficiently is enabled by the binding or mixing of the substance which exhibits affinity for phosphatidyl serine in the presence of serum albumin to the desired physiologically active substance, and by accumulating the physiologically active substance to the target cell.

The phospholipid which serves the target molecule in the accumulation of the physiologically active substance, which is preferably a nucleic acid, and the vector containing the peptide of the present invention is a molecule which constitutes the cell membrane of the cell, and the cell membrane is ubiquitously found in every cell with no exception. This is radically different from the molecule that served the target molecule in conventional targeting wherein the target molecule was a molecule such as a particular antigen, a particular receptor comprising a peptide, or a particular molecular which was been dispersed on the cell membrane and which was not necessarily found in every cell. In other words, every cell as well as every tissue and organ constituted from any cell is a candidate for introduction of the physiologically active substance, which is preferably a nucleic acid, by using the vector of the present invention. If the affinity imparted by the carrier is the affinity for a particular phospholipid, function of the physiologically active substance, which is preferably a nucleic acid, can be selectively developed in particular cell, in particular tissue, or in particular organ out of every cell or every tissue or organ constituted from the cell.

As described above, the delivery method according to the fifth aspect of the present invention is a drug delivery method or a drug delivery system wherein a drug or a reagent is delivered into the interior of the cell on the basis of an entirely new concept that, by using phosphatidyl serine as the target phospholipid molecule, a physiologically active substance, which is preferably a nucleic acid, can be selectively accumulated at the surface layer of an abnormal cell, tissue or organ to thereby promote introduction of the substance into the cell and to thereby enhance the action and effects of the substance.

Phosphatidyl ethanolamine is another phospholipid found in the outer layer of the lipid bilayer in the cell membrane, whose content increases in an abnormal cell as described above, for example, in an injured, denatured, or activated cell. The drug delivery method or the drug delivery system may be conducted by using the phosphatidyl ethanolamine for the target molecule as in the case of the method wherein phosphatidyl serine is used for the target molecule.

As described in the section of the vector of the present invention, introduction of the substance into the desired particular cell or organ at a high efficiency is enabled by increasing the amount of phosphatidyl serine translocated to the surface of such cell or organ by the action of a drug in advance. To be more specific, amount of the phosphatidyl serine translocated to the surface of the particular cell or organ increases when a drug is reacted with the particular cell or organ, and introduction of the substance into the cell or organ at an increased efficiency by the delivery method according to the fifth aspect of the present invention is thereby enabled. For example, in the case of treating cancer by chemotherapy, amount of phosphatidyl serine translocated to the surface of the tumor cell can be increased by administering an anticancer drug, and introduction into the tumor cell of the substance exhibiting anti-tumor effects by the delivery method according to the fifth aspect of the present invention is thereby enabled to realize an improved therapeutic effect.

An exemplary such reagent is an anticancer drug such as doxorubicin.

An exemplary procedure which may be used with the method of the present invention comprises the steps of
(1) administering the patient with a drug which increases phosphatidyl serine in the cell surface;
(2) administering the patient with the gene and the vector of the present invention; and
(3) before administering the gene and the vector of the present invention, optionally confirming the increase of the phosphatidyl serine in the cell surface, for example, by administering the peptide of the present invention which has been labeled with a radioisotope such as technetium-99m to thereby detect the cell or the tissue wherein phosphatidyl serine has increased in the cell surface.

### EXAMPLES

Next, the present invention is described in further detail by referring to Examples which are presented by way of illustration and which by no means limit the scope of the invention. The abbreviations used in the following description are those based on custom abbreviations used in the art.

**Table 1(1)**

| Disease | Gene, antisense, or decoy introduced in the cell |
|---|---|
| Cancer | interleukin 2 gene, interleukin 4 gene, interleukin 10 gene, interleukin 18 gene, interferon α gene, interferon β gene, interferon γ gene, Fas ligand gene, TNF α (Tumor necrosis factor α) gene, TRAIL (TNF-related apoptosis-inducing ligand) gene, I κB α gene, p53 gene, p21 gene, p 16^{INK4a} gene, caspase 3 gene, caspase 8 gene, BAX (Bcl-2-associated X protein) gene, FADD (Fas associated death domain protein) gene, GM-CSF (granulocyte-macrophage colony-stimulating factor) gene, HSV-tk (herpes simplex virus thymidine kinase) gene, SPF4 (platelet factor 4) gene, cytosine deaminases gene, E. coli uracil phosphoribosyltransferase, ABC transporter gene, MDR 1 (multidrug resistance) gene, K-ras antisense, ICAM-1 (intercellular adhesion molecule-1) gene, ICAM-2 gene, HTLV-I tax (Human T-Cell leukemia virus 1 tax) gene, HGF (Hepatocyte growth factor) gene, p51 gene, p63 gene, mutant FGF (Fibroblast growth factor) receptor gene, MIP-1β (macrophage inflammatory protein-1β) gene, CD40 ligand gene, Gelsolin gene, IGF-1 (Insulin-like growth factor-1) gene, adenovirus E1 gene, BCL-2 antisense |
| Articular rheumatism | soluble TNF α receptor gene, soluble interleukin 6 receptor gene, interleukin 4 gene, interleukin 13 gene, p21 gene, interleukin 1 receptor antagonist gene, Fas ligand gene |

**Table 1(2)**

| Disease | Gene, antisense, or decoy introduced in the cell |
|---|---|
| Bronchial asthma | mite antigen gene |
| Arteriosclerosis obliterans | VEGF (Vascular endothelial growth factor) gene, HGF gene, Angiopoietin-1 gene |
| Arterial sclerosis | NOS (Nitric oxide syntheses) gene |
| Angina pectoris | VEGF (Vascular endothelial growth factor) gene, HGF gene |
| Restenosis after PTCA | E2F decoy, NFκB decoy, CDC2 kinase antisense, c-myc antisense |
| Ischemic reperfusion injury | FGF-1 gene, NGF (Nerve growth factor) gene, GDNF (Glial cell-derived neurotrophic factor) gene |
| HSV infection | HSV antisense |
| HIV infection | HIV envelope gene, Tar decoy, RRE decoy |
| Viral hepatitis | interferon α gene, interferon β gene, HBV envelope protein gene, HBV antibody gene |
| Various infections | MDC (Macrophage-derived chemokine) gene |
| Infectious shock, sepsis, ARDS | NFκB decoy, soluble TNF α receptor gene |

**Table 1(3)**

| Disease | Gene, antisense, or decoy introduced in the cell |
|---|---|
| GVHD | Fas ligand gene |
| Primary pulmonary hypertension | mutant MCP-1 (Monocyte chemoattractant protein 1) gene |
| Diabetic retinopathy | SPF4 gene |
| Aneurysm | NFκB decoy, Ets-1 decoy |
| Muscular atrophy | Bcl-2 gene |
| Inflammatory colitis, Crohn's disease | soluble TNF α receptor gene, HGF gene, SOD (Superoxide dismutase) gene |
| α1-antitrypsin deficiency | α1-antitripsin gene |
| Familial hypercholesterolemia | LDL (low density lipoprotein) receptor gene |
| Hyperlipoproteinia | apolipoprotein a antisense |

### Example 1: Chemical synthesis of the peptide

Peptides having the amino acid sequences of SEQ ID NO:1 to SEQ ID NO:27 were synthesized by solid phase synthesis by using an automatic peptide synthesizer (432A, manufactured by Applied Biosystems). It is to be noted that, in synthesizing a peptide having a length of 30 residues or longer, synthesis was suspended without deprotecting the 25th amino acid residue, and the synthesis was accomplished by resuming the synthesis after providing the synthesizer with the amino acid columns of 26th and remaining residues. Unless otherwise noted, the synthesis was conducted in accordance with the manufacturer's manual. The peptide was cleaved, deprotected, precipitated in ether, stripped of the ether, dissolved in distilled water, and lyophilized. Next, the peptides of SEQ ID NO:1 to SEQ ID NO:26 were dissolved in 20% acetonitrile aqueous solution containing 10mM HCl, and the peptide of SEQ ID NO:27 was dissolved in 15% acetonitrile/15% isopropanol aqueous solution containing 10mM HCl. By using C18 column (CAPCELLPAK C18AG120, manufactured by Shiseido) and high performance liquid chromatography (625 LC System, manufactured by Waters), the peptides of SEQ ID NO:1 to SEQ ID NO:26 were purified so that single peak is obtained in linear concentration gradient of 20% to 70% acetonitrile aqueous solution containing 10mM HCl, and the peptide of SEQ ID NO:27 was purified so that single peak is obtained in linear concentration gradient of 15% to 50% acetonitrile/15% to 50% isopropanol aqueous solution containing 10mM Hcl. The thus purified peptides were lyophilized, dissolved in distilled water, and stored after frozen.

The peptides were produced at a yield of 30 mg to 40 mg, respectively.

### Example 2: Assay of peptide (1)

The resulting synthetic peptides were evaluated for their molecular weight by mass spectroscopy using MALDI-TOFMS mass spectrometer (VoyagerDE-STR, manufactured by PE Biosystems) to thereby confirm that the resulting peptides were the desired peptides. Unless otherwise noted, the spectroscopy was conducted in accordance with the manufactuler's manual. The procedure was as summarized below. First, α-cyano-4-hydroxycinnamic acid (CHCA) was dissolved in 0.1 vol% TFA/50 vol% acetonitrile/pure water to prepare a 10 mg/mL matrix solution. Then, 0.5 µL of aqueous solution (10 pmol/µL) of the peptide produced by the procedure described in Example 1 and 0.5 µL of the matrix solution were mixed on a sample plate, and the mixture was dried for crystallization of the sample. The analysis was conducted under the following conditions.
Measurement mode: Linear, positive
Calibration: external standard method (Note that the standards were (i) Angiotensin I, (ii) ACTH (1-17clip), (iii) ACTH (18-39clip), (iv) ACTH (7-38clip), and (v) Insulin (bovine).)

It was then confirmed that all of the synthetic and purified peptides were consistent with the theoretical molecular weight.

### Example 3: Assay of peptide (2)

The solutions of the synthetic peptide produced in Example 1 were determined for their concentration by analyzing amino acid composition by ninhydrine method. The samples were exsiccated in a glass test tube, and after adding 100 µL of 6N HCl and evacuating and sealing the test tube, hydrolysis was allowed to proceed at 110°C for 22 hours. The samples were then exsiccated, dissolved in pure water, and analyzed in an amino acid analyzer (L-8500, manufactured by HITACHI). The peptide aqueous solutions had a concentration of 7 to 10 mg/mL.

### Example 4: Measurement of turbidity in BSA

Aqueous solutions were prepared so that the resulting solution had a bovine serum albumin (BSA) concentration of 1% and the peptide concentration of 50 µM. The solutions were evaluated for their absorbance at a wavelength of 600 nm by using a spectrophotometer (DU640, manufactured by Beckman). The results are shown in Table 2. No sample exhibited an absorbance that exceeded 0.1.

**Table 2**

| SEQ ID NO: | OD 600 |
|---|---|
| SEQ ID NO:1 | 0.03 |
| SEQ ID NO:2 | 0.01> |
| SEQ ID NO:3 | 0.01> |
| SEQ ID NO:5 | 0.01> |
| SEQ ID NO:6 | 0.01> |
| SEQ ID NO:7 | 0.01> |
| SEQ ID NO:8 | 0.01> |
| SEQ ID NO:10 | 0.01> |
| SEQ ID NO:11 | 0.01> |
| SEQ ID NO 15 | 0.01> |
| SEQ ID NO:16 | 0.01> |
| SEQ ID NO:17 | 0.01> |
| SEQ ID NO:19 | 0.01> |
| SEQ ID NO:23 | 0.01> |

### Example 5: Measurement of CD spectrum

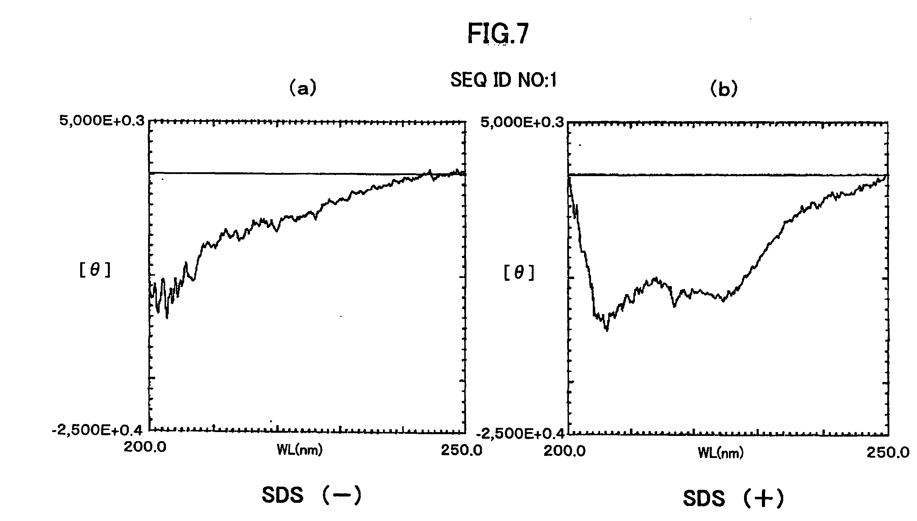

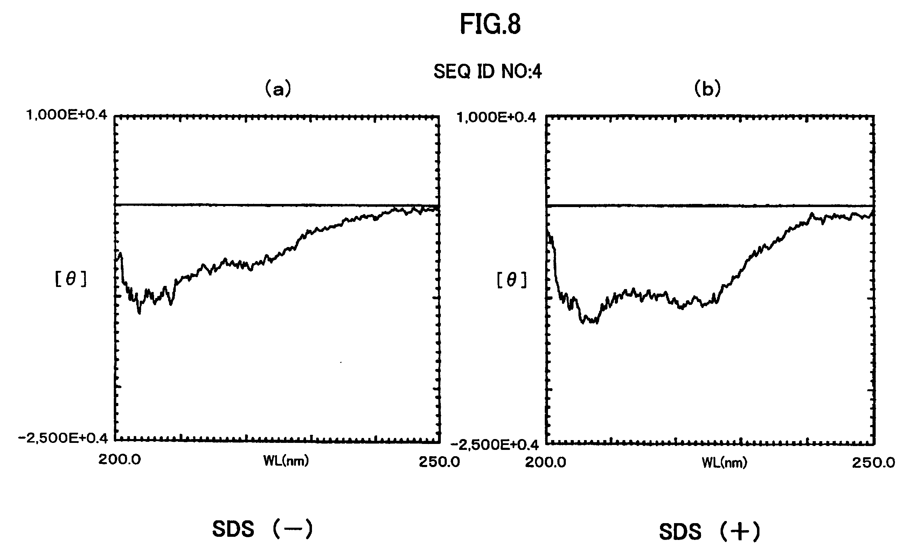

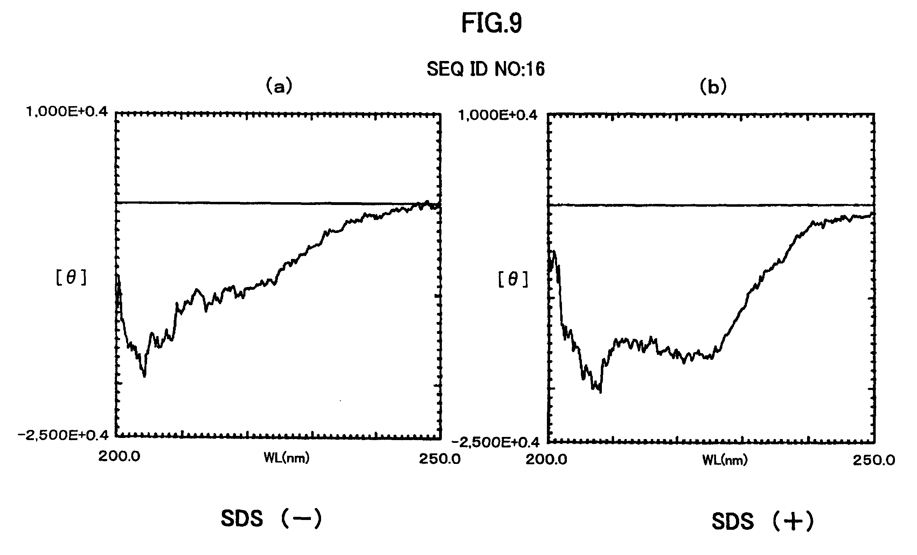

The peptide dissolved in 10mM phosphate buffer (pH = 7) containing 50mM NaCl was evaluated for its CD spectrum in the presence and in the absence of 5mM SDS by using a circular dichroism spectrophotometer (J-500A, manufactured by JASCO). The cell length was 1 mm. The measurement was conducted at 35°C for 6 times in total. FIGS. 7 to 9 are respectively views showing mean residue ellipticity in CD spectroscopy of the peptide of SEQ ID NO:1, the peptide of SEQ ID NO:4 and the peptide of SEQ ID NO:16. As evident in FIGS. 7 to 9, the mean residue ellipticity obtained in the presence of SDS was the so called W curve and local minimums were found in the areas at the wavelength of 205 to 210 nm and 220 to 225 nm, and α-helix structure was thereby confirmed. In other words, it was revealed that the peptides of SEQ ID NO:1, SEQ ID NO:4, and SEQ ID NO:16 showing the gene-introducing ability are in α-helix structure in the presence of SDS while they do not take α-helix structure in an aqueous solution solely containing an inorganic salt.

### Example 6: Synthesis of oligonucleotide

Unlabeled 21mer oligonucleotide was prepared by purification using OPC column (manufactured by Applied Biosystems). FITC-labeled 21mer oligonucleotide was prepared by HPLC using reverse phase chromatography. Synthesis of the oligonucleotide was consigned to Sawady Technology Co., Ltd.

### Example 7: Preparation of plasmid

For the expression plasmid including firefly luciferase gene as the reporter gene, there were used a commercially available plasmid (pGL3-Control Vector, manufactured by Promega) wherein firefly luciferase gene had been incorporated under SV40 early promoter, a plasmid (pCMV-Luc(F)) wherein firefly luciferase gene had been incorporated under CMV early promoter, and a plasmid (pEF-Luc) wherein firefly luciferase gene had been incorporated under EF-1 α promoter (a plasmid (pEF-tk) wherein HSV-tk gene had been incorporated under FEF-1 α promoter). For the expression plasmid including HSV-tk gene as the reporter gene, there were used a plasmid (pEF-tk) wherein HSV-tk gene had been incorporated under EF-1 α promoter, and a plasmid (pCMV-tk) wherein HSV-tk gene had been incorporated under CMV early promoter. These plasmids and pUC119 and pBR322 which are respectively a universal plasmid were amplified in E. coli when necessary, and purified by a known method before use.

### Example 8: Evaluation of nucleic acid-binding ability

### (1) Evaluation of binding ability to oligonucleotide

The 21mer oligonucleotide prepared by the procedure described in Example 6 (final concentration, 6.7 µM) and the peptide prepared by the procedure described in Examples 1 to 3 were mixed in 20mM Tris-HCl buffer (pH = 7.2) containing 150mM NaCl at a charge ratio (+/- ratio) of 0 to 10, and the mixture was allowed to stand at 37°C for 30 minutes. Next, 7.5 µL of this solution was mixed with an equal amount of Tris-borate buffer (pH = 8.2) containing 80% formamide, and electrophoresis was conducted on 25% polyacrylamide gel containing 7M urea. After the electrophoresis, the gel was stained with 5% aqueous solution of Stains all (manufactured by Funakoshi) containing 50% formamide and washed with water to thereby determine the binding of the oligonucleotide and the peptide.

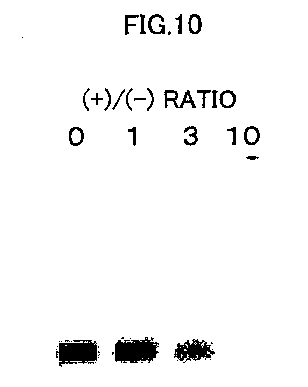

FIG. 10 shows the electrophoregram for the peptide of SEQ ID NO:1. The +/- ratio (charge ratio) indicated in FIG. 10 is the ratio of the number (+) of the positively-charged groups in the peptide to the number (-) of negatively-charged groups in the nucleic acid. As evident in FIG. 10, the amount of oligonucleotide that became bound to the peptide increased with the increase in the charge ratio (+/- ratio), and the oligonucleotide was fully bound to the peptide at a charge ratio (+/- ratio) of 10. The peptides of SEQ ID NO:2 to SEQ ID NO:24 were also found to bind at a charge ratio (+/- ratio) of 10.

### (2) Evaluation of binding ability to plasmid

The plasmid prepared by the procedure described in Example 7 (pUC119; final concentration, 20 µg/mL) and the peptides prepared by the procedure described in Examples 1 to 3 were mixed in 20mM Tris HCl buffer (pH = 7.2) containing 150mM NaCl at a charge ratio (+/- ratio) of 0 to 3, and the mixture was allowed to stand at 37°C for 30 minutes. Electrophoresis was then conducted on 1% agarose gel to thereby determine the binding of the plasmid and the peptide.

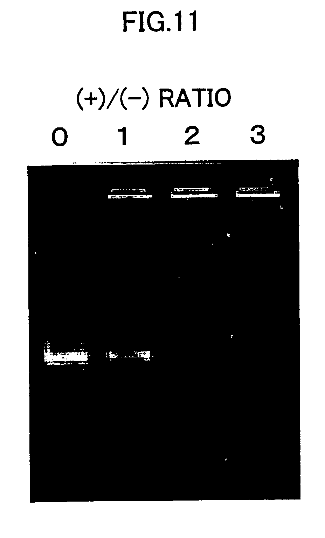

FIG. 11 shows the electrophoregram for the peptide of SEQ ID NO:1. The +/- ratio (charge ratio) indicated in FIG. 11 is the ratio of the number (+) of the positively-charged groups in the peptide to the number (-) of negatively-charged groups in the plasmid. As evident in FIG. 11, the amount of plasmid that became bound to the peptide increased with the increase in the charge ratio (+/- ratio), and the plasmid was fully bound to the peptide at a charge ratio (+/- ratio) of 3. The peptides of SEQ ID NO:2 to SEQ ID NO:24 were also found to bind at a charge ratio (+/- ratio) of 3.

### Example 9: Evaluation of nucleic acid-introducing ability (1)

A cell line established from monkey kidney (Vero cell) and a human bladder cancer cell (T24 cell) purchased from American Type Culture Collection (ATCC) and human lung cancer cell (A549 cell) purchased from Dainippon Pharmaceutical were inoculated on a 24 well culture plate (manufactured by NALGEN NUNC) at 1 x 10⁵ cells/well. Vero cell and A549 cell were cultivated in DMEM (manufactured by Life Technologies Oriental) supplemented with 10% fetal bovine serum (hereinafter referred to as FBS, manufactured by NICHIREI), and T24 cell was cultivated in McCoy' s 5a (manufactured by Life Technologies Oriental) supplemented with 10% FBS in 5% CO₂ atmosphere at 37°C for 24 hours. After removing the medium, opti-MEM (manufactured by Life Technologies Oriental) prepared to have the concentration of the luciferase-expressing plasmid prepared in Example 7 of 1 µg/ mL and the concentration of the peptide prepared in Example 1 of 1.25, 2.5, or 5 µM was added, and the cells were cultivated for 5 hours. Next, in the case of Vero cell and A549 cell, the culture medium was replaced with DMEM supplemented with 10% FBS, and in the case of T24 cell, the medium was replaced with McCoy's 5a supplemented with 10% FBS, and cultivation was continued in 5% CO₂ atmosphere and at 37°C for another 24 hours. The luciferase activity expressed in the cell was then measured by the method instructed in luciferase assay system (manufactured by Promega). To be more specific, the cells were washed with phosphate-buffered saline (hereinafter referred to as PBS, manufactured by SIGMA), and the cells were lyzed with Passive Lysis Buffer attached to the kit. 20 µL of this cell lysate and 100 µL of the Luciferase Assay Reagent II attached to the kit was added to a fluorescence-measuring plate (Microlite™ 1 plate, manufactured by Dynatech), and after mixing, fluorescence was measured for 1 second by using a multilabel counter (ARVO™ SY1420 MULTILABEL COUNTER, manufactured by Wallac Beltold.

Concentration of the protein in the cell lysate was measured by mixing 8 µL of the cell lysate and 200 µL of the protein assay solution (manufactured by Biorad) with 792 µL of ultrapure water in a disposable cuvette (UV fluorescent cuvette A204X, manufactured by Funakoshi), allowing the mixture to stand for 5 minutes at room temperature, and measuring the absorption at 595 nm with a spectrophotometer (DU640, manufactured by Beckman). Bovine serum albumin (BSA) was used for the standard protein. By using the thus obtained results, count per 1 second per 1 mg of protein of the cell lysate was calculated to thereby use the count as the luciferase activity, and the highest count in the three conditions of different peptide concentration was designated the gene-introducing ability of the peptide.

### Example 10: Evaluation of nucleic acid-introducing ability (2)

Vero cell was inoculated in the well of a chamber slide (Lab-Tek II chamber slide, size 4 well, manufactured by NALGEN NUNC) at a rate of 1 x 10⁵ cells/well, and the cells were cultivated in DMEM supplemented with 10% FBS in 5% CO₂ atmosphere at 37°C for 24 hours. After removing the medium, opti-MEM containing 300 nM FITC-labeled oligonucleotide prepared in Example 6 and 2.5 µM of the peptide of SEQ ID NO:1 prepared in Example 1 was added, and the cultivation was continued for 4 hours. The medium was replaced with DMEM supplemented with 10% FBS, and the cultivation was continued in 5% CO₂ atmosphere at 37°C for another 24 hours. After washing the cell with PBS, the cells were fixed by using PBS containing 4% paraformaldehyde, and accumulation of FITC-labeled oligonucleotide in the cell nucleus was confirmed by using a fluorescence microscope (AH-3, manufactured by Olympus). It was then confirmed that FITC-labeled oligonucleotide was accumulated in the cell nucleus. On the other hand, accumulation of FITC-labeled oligonucleotide in the cell was not confirmed in the absence of the peptide of SEQ ID NO:1.

### Example 11: Evaluation of nucleic acid-introducing ability into cell of the peptide (1)

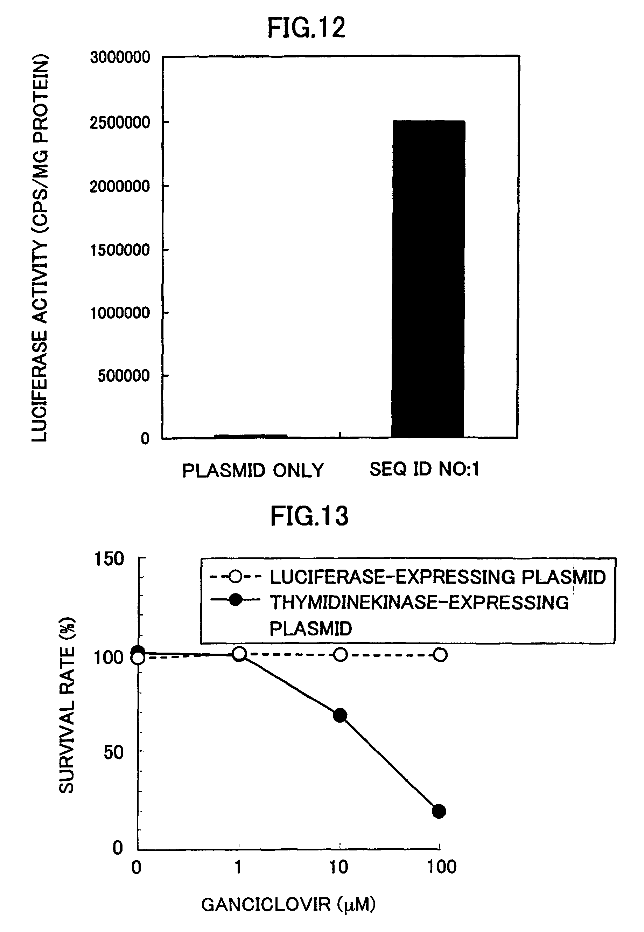

The peptide of SEQ ID NO:1 was evaluated for its ability of introducing the nucleic acid into a cell by the procedure described in Example 9. FIG. 12 is a view wherein difference in the amount of plasmid introduced into the cell is compared in the presence and absence of the peptide of SEQ ID NO:1 by using luciferase activity. As shown in FIG. 12, the peptide of SEQ ID NO:1 was found to have the ability of introducing the nucleic acid into a cell, while the nucleic acid was not introduced in the absence of the peptide of SEQ ID NO:1.

### Example 12: Evaluation of nucleic acid-introducing ability into cell of the peptide (2)

The peptides of SEQ ID NO:2 to SEQ ID NO:18 having a sequence wherein one or two locations in "the amino acid sequence of 18 amino acids exhibiting the four sided structure of the present invention" have been substituted in the peptide of SEQ ID NO:1 was evaluated for their ability of introducing the nucleic acid into a cell by the procedure described in Example 9. It was then found that all peptides had the ability of introducing the nucleic acid into a cell.

It is to be noted that the nucleic acid-introducing ability was classified as described below on the bases of the fluorescent count per 1 mg of protein per 1 second (cps/mg protein). The results are listed in Table 3.

| | |
|---|---|
| 10,000 to less than 100,000 | 1+ |
| 100,000 to less than 1,000,000 | 2+ |
| 1,000,000 to less than 10,000,000 | 3+ |
| 10,000,000 or more | 4+ |
| 100,000,000 to less than 1,000,000,000 | 5+ |
| 1,000,000,000 or more | 6+ |

It is to be noted that the nucleic acid-introducing ability of SEQ ID NO:1 was 3+ in the above classification.

**Table 3**

| SEQ ID NO: | Nucleic acid-introducing ability |
|---|---|
| SEQ ID NO:2 | 3+ |
| SEQ ID NO:3 | 2+ |
| SEQ ID NO:4 | 4+ |
| SEQ ID NO:5 | 2+ |
| SEQ ID NO:6 | 2+ |
| SEQ ID NO:7 | 2+ |
| SEQ ID NO:8 | 3+ |
| SEQ ID NO:9 | 3+ |
| SEQ ID N0:10 | 3+ |
| SEQ ID NO:11 | 3+ |
| SEQ ID NO:12 | 2+ |
| SEQ ID NO:13 | 3+ |
| SEQ ID NO:14 | 3+ |
| SEQ ID NO:15 | 2+ |
| SEQ ID NO:16 | 4+ |
| SEQ ID NO:17 | 2+ |
| SEQ ID NO:18 | 4+ |
| SEQ ID NO:19 | 4+ |
| SEQ ID NO:20 | 4+ |
| SEQ ID NO:21 | 2+ |
| SEQ ID NO:22 | 3+ |
| SEQ ID NO:23 | 2+ |
| SEQ ID NO:24 | 1+ |

### Example 13: Evaluation of nucleic acid-introducing ability into cell of the peptide (3)

The peptides of SEQ ID NO:19 to SEQ ID NO:22 having a sequence wherein three locations in "the amino acid sequence of 18 amino acids exhibiting the four sided structure of the present invention" had been substituted in the peptide of SEQ ID NO:1 was evaluated for their ability of introducing the nucleic acid into a cell by the procedure described in Example 9. It was then found that these peptides had the ability of introducing the nucleic acid into a cell.

It is to be noted that the nucleic acid-introducing ability was classified as described in Example 12 on the bases of the fluorescent count per 1 mg of protein per 1 second. The results are listed in Table 3.

### Example 14: Evaluation of nucleic acid-introducing ability into cell of the peptide (4)

The peptides of SEQ ID NO:23 and SEQ ID NO:24 which are respectively a deletion mutant of the peptides of SEQ ID NO:1 and SEQ ID NO:4 were prepared, and these peptides were evaluated for their ability of introducing the nucleic acid into a cell by the procedure described in Example 9. It was then found that all of the peptides had the ability of introducing the nucleic acid into a cell.

It is to be noted that the nucleic acid-introducing ability was classified as described in Example 12 on the bases of the fluorescent count per 1 mg of protein per 1 second. The results are listed in Table 3.

### Example 15: Evaluation of nucleic acid-introducing ability into cell of the peptide (5)

The peptide of SEQ ID NO:16 was evaluated for its ability of introducing the nucleic acid into various cancer cell lines by the procedure described in Example 9.

The cancer cell used were human uterine cancer cell, MES-SA/Dx5 (purchased from ATCC), a transformant cell from human kidney 293 (purchased from ATCC), human hepatoma cell, SK-HEP-1 (purchased from ATCC), human ovarian cancer cell, SK-OV-3 (purchased from ATCC), rat brain tumor cell, F98 (purchased from ATCC), mouse melanoma cell, B16/BL6 (provided by Chemistry Division, Institute of Immunological Science, Hokkaido University), human uterine cancer cell, MES-SA (purchased from ATCC), mouse lung cancer cell, Lewis Lung Carcinoma (provided by Japanese Foundation for Cancer Research), mouse hapatoma cell, MH134 (provided by Central Laboratories for Experimental Animals), human uterine cancer cell, MES-SA/Mx2 (purchased from ATCC), human medulloblastoma cell, Daoy (purchased from ATCC), human uterine cervix cancer cell, HeLa (purchased from ATCC), human breast cancer cell, MCF7 (purchased from ATCC), human glioblastoma cell, U-87 MG (purchased from ATCC), human breast cancer cell, MDA-MB-468 (purchased from ATCC), human renal cancer cell, A-498 (purchased from ATCC), mouse melanoma cell, B16/F10 (purchased from ATCC), human prostatic cancer cell, DU 145 (purchased from ATCC), human brain tumor cell, U-138 MG (purchased from ATCC) and mouse sarcoma cell, Meth-A (provided by National Cancer Center). The culture media used for the propagation of the cells are indicated in Table 5. It is to be noted that, of the additives, NEAA (manufactured by ICN) is a nonessential amino acid, Na·Pyr (manufactured by ICN) is sodium pyruvate. The FBS used were the one manufactured by NICHIREI in all cases, and the culture media were those manufactured by Life Technologies Oriental. All propagation media were supplemented with penicillin/streptomycin (manufactured by ICN).

Subcultured cells were inoculated on 24 well culture plate at a rate of 1 x 10⁵ cells/well, and the cells were cultivated in the respective culture medium in 5% CO₂ atmosphere at 37°C for 24 hours. After removing the medium, opti-MEM prepared such that the luciferase-expressing plasmid concentration prepared in Example 7 was 1 µg/mL and the peptide concentration prepared in Example 1 was 2.5 µM was added, and the cultivation was continued for 5 hours. The medium was then replaced with the respective propagation medium, and the cultivation was continued in 5% CO₂ atmosphere at 37°C for another 24 hours. Luciferase activity expressed in each cell was then measured by the procedure described in Example 9, and the gene-introducing ability of the peptides is indicated in Table 4 in accordance with the criteria described in Example 12.

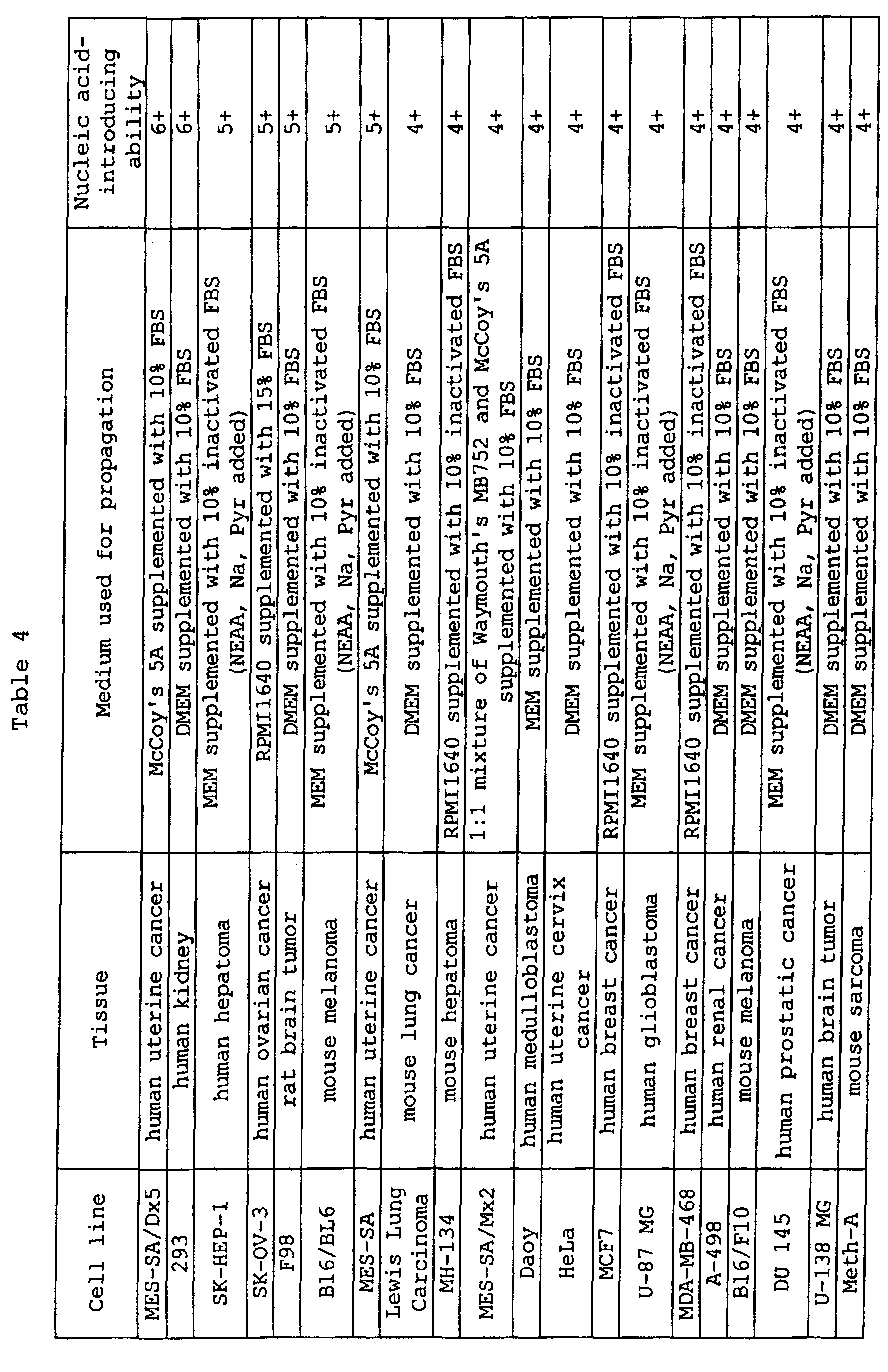

### Example 16: Evaluation of nucleic acid-introducing ability into cell of the peptide (6)

The HSV-tk-expressing plasmid prepared in Example 7 was introduced in Lewis lung carcinoma cell (mouse lung cancer cell) by using the peptide of SEQ ID NO:16 prepared in Example 1 to thereby evaluate the effect of increasing the sensitivity for ganciclovir (hereinafter referred to as GCV). To be more specific, cells were inoculated in 96 well culture plate (manufactured by NALGEN NUNC) at a rate of 5 x 10³ cells/well, and cultivated in DMEM supplemented with 10% FBS in 5% CO₂ atmosphere at 37°C for 24 hours. After removing the culture medium, 100 µL of opti-MEM prepared to have a concentration of the HSV-tk-expressing plasmid of 1 µg/mL and a concentration of the peptide of SEQ ID NO:16 of 2.5 µM was added to each well, and the cultivation was continued for 5 hours. Next, 100 µL of the DMEM supplemented with 20% FBS respectively containing 0, 2, 20, and 200 µM of GCV (Denosine, manufactured by Tanabe Seiyaku) was added to each well, and cultivation was continued in 5% CO₂ atmosphere at 37°C for 3 days. The effect of suppressing the cell propagation was measured by WST-1 assay by adding 10 µL of WST-1 solution (manufactured by Takara Shuzo) in each well, allowing the reaction to proceed for 1 hour, and measuring the absorbance at a wavelength of 620 nm with a multi label counter by using a reference wavelength of 450 nm. As shown in FIG. 13, the cell propagation was found to be suppressed in the case of the Lewis lung carcinoma cell having the HSV-tk-expressing plasmid introduced therein in a manner dependent on the dose of the GCV whereas the effect of increasing the GCV sensitivity was not found in the control cell having the luciferase-expressing plasmid introduced therein.

### Example 17: Evaluation of storage stability of the complex with nucleic acid

Storage stability of the complex of the plasmid and the peptide was evaluated.

Luciferase-expressing plasmid was introduced in Vero cell in accordance with the procedure described in Example 9 by using the opti-MEM prepared to have a concentration of the luciferase-expressing plasmid prepared in Example 7 of 2 µg/mL and a concentration of the peptide of SEQ ID NO:4 prepared in Example 1 of 5 µM to thereby measure the luciferase activity. The opti-MEM containing plasmid and peptide used were the one which had been prepared immediately before the gene introduction, and the one which had been prepared 1 week (7 days) in advance and stored at 4°C.

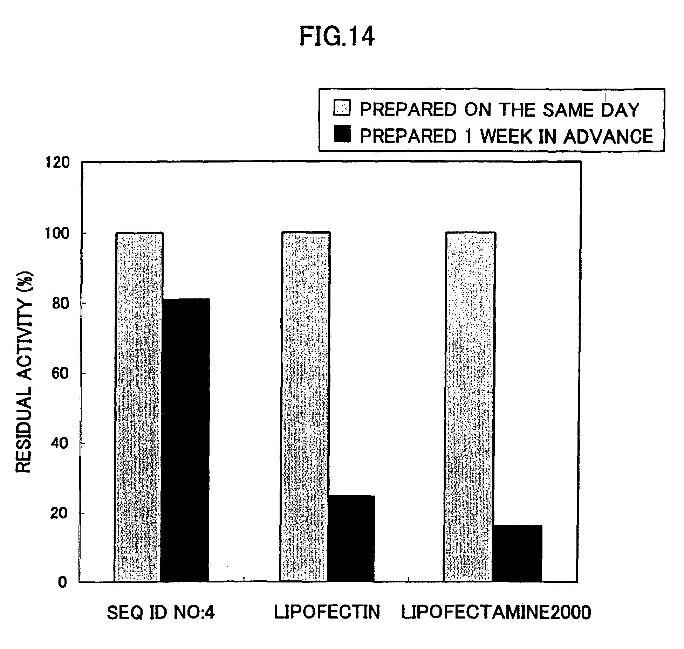

FIG. 14 shows gene-introducing activity of the peptide/plasmid complex stored at 4°C for 1 week as a value in relation to the gene-introducing activity of the peptide/plasmid complex prepared immediately before the gene introduction which is assumed to be 100%. As shown in FIG. 14, the gene-introducing activity of the peptide/plasmid complex stored at 4°C for 1 week was substantially equivalent to that of the gene-introducing activity of the peptide/plasmid complex prepared immediately before the gene introduction.

For the purpose of reference, gene-introducing activity of Lipofectin and LipofectAMINE 2000 (manufactured by Life Technologies Oriental), which are commercially available plasmid-introducing agents, were also evaluated by mixing these reagent with luciferase-expressing plasmid in accordance with the attached protocol, and evaluating their gene-introducing activity after storing at 4°C for 1 week. Both Lipofectin and LipofectAMINE 2000 exhibited a marked decrease in the gene-introducing activity. The results are also shown in FIG. 14.

### Example 18: Evaluation of nuclease resistance-imparting ability (1)

Nuclease resistance-imparting ability for natural (P=O form) oligonucleotide was evaluated. To be more specific, 2 U of nuclease Bal 31 (manufactured by Takara Shuzo) was added to 20mM Tris-HCl buffer (pH = 8) containing 50 µM of the peptide of SEQ ID NO:1 prepared in Example 1, 3 µM of P=O form oligonucleotide prepared in Example 6, 150mM NaCl, 12mM MgCl₂, and 12mM CaCl₂, and the reaction was allowed to take place at 30°C for 60 minutes. Next, the oligonucleotide which escaped from the decomposition was extracted with phenol/chloroform, and subjected to electrophoresis by the procedure described in Example 8 to stain the oligonucleotide.

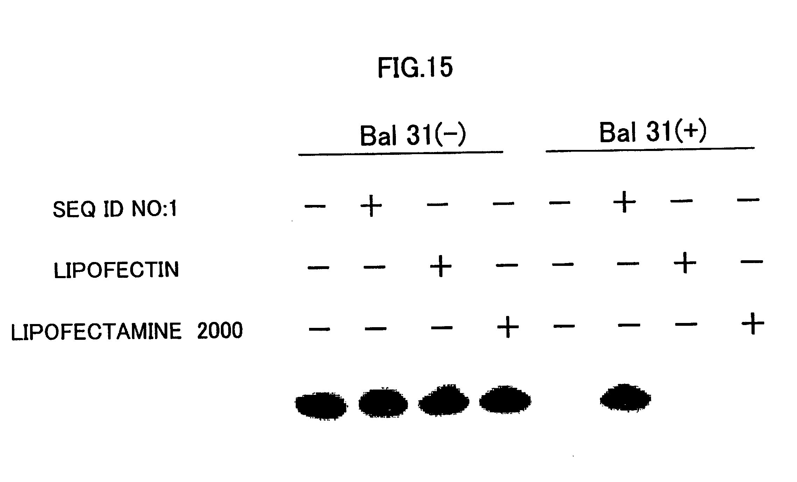

FIG. 15 shows the electropherogram. As shown in FIG. 15, the peptide of SEQ ID NO:1 was found to exhibit nuclease resistance-imparting ability for the natural (P=O form) oligonucleotide.

For the purpose of reference, nuclease resistance-imparting ability was also evaluated for lipofectin and LipofectAMINE 2000, which are commercially available plasmid-introducing agents. Both lipofectin and LipofectAMINE 2000 exhibited no stained oligonucleotide in the electrophoregram, indicating the decomposition of the oligonucleotide. The results are shown in FIG. 15.

### Example 19: Evaluation of nuclease resistance-imparting ability (2)

pBR322 prepared by the procedure described in Example 7 (final concentration, 20 µg/mL) and the peptide of SEQ ID NO:16 prepared in Example 1 were mixed in 20mM Tris-HCl buffer (pH = 8) containing 150mM NaCl and 1.7mM MgCl₂ at a charge ratio (+/- ratio) of 0 to 10, and 5 U of DNase I (manufactured by Takara Shuzo) was then added. After allowing the reaction to proceed at 30°C for 60 minutes, the plasmid which escaped the decomposition was extracted with phenol/chloroform, and subjected to electrophoresis by the procedure described in Example 8 to stain the plasmid.

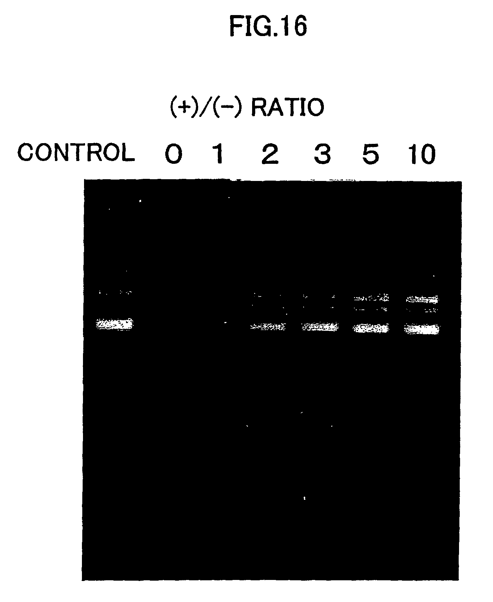

FIG. 16 shows the electropherogram. As shown in FIG. 16, the peptide of SEQ ID NO:16 was found to exhibit nuclease resistance-imparting ability for the plasmid.

### Example 20: Evaluation of the affinity of the peptide for phosphatidyl serine (EIA)

The specific affinity of the peptide for phosphatidyl serine was evaluated by measuring activity of the peptide for inhibiting the binding of the human Factor VIII to phosphatidyl serine by using human Factor VIII which is known to bind to phosphatidyl serine but not to phosphatidyl choline in the presence of serum albumin. To be more specific, 100 µL of ethanol solution containing 10 µg/mL of phospholipids at a phosphatidyl serine (manufactured by SIGMA): phosphatidyl choline (manufactured by SIGMA) ratio of 3:7 was added to the wells of a 96 well plate (Immulon I, manufactured by Dynatech), and exsiccated by using a centrifugal evaporator (EC-95C, manufactured by Sakuma Seisakusho) at 40°C for 40 minutes. 200 µL each of TBS (10mM Tris-HCl (pH 7.4) containing 1% bovine serum albumin (BSA Fraction V, hereinafter referred to as BSA, manufactured by Seikagaku Corporation) and 0.9% (W/V) NaCl solution were added to each well, and the wells were blocked by allowing the plate to stand at 37°C for 2.5 hours. After washing the plate with water, 100 µL of TBS solution supplemented with 5% BSA mixed with human Factor VIII (manufactured by American Diagnostica) at a final concentration 1 µg/mL and the peptide of predetermined dose prepared in Example 1 was added to each well, and the reaction was allowed to take place at 4°C for 24 hours.

After the completion of the reaction, the human Factor VIII that became bound to the phosphatidyl serine was measured by enzyme immunoassay (EIA) in accordance with the general procedure described in a book ("Enzyme Immunoassay (3rd ed.)", Eiji Ishikawa et al., Igaku-Shoin, 1987). To be more specific, the measurement was conducted by using anti-human Factor VIII mouse monoclonal antibody (ESH8, manufactured by American Diagnostica) for the primary antibody, horseradish peroxidase-labeled anti-mouse IgG antibody (P0260, manufactured by Daco) for the secondary antibody, and tetramethylbenzidine for the chromogenic substrate, and measuring the absorption at a wavelength of 450 nm by using a reference wavelength of 630 nm on a spectrophotometer (NJ-2100, manufactured by Intermed).

Intensity of the affinity of the peptide for the phosphatidyl serine was evaluated by using the value obtained by subtracting the absorption of the well having no human Factor VIII added thereto from the absorption of the well having only the human Factor VIII added thereto as the control value (100%), and designating the peptide concentration at which the value 0.5 was obtained when the value obtained by subtracting the absorption of the well having only the peptide added thereto from the absorption of the well having a mixture of the human Factor VIII and the peptide added thereto were divided by the control value as the IC₅₀ value, and evaluating the intensity to be ++ when the IC₅₀ value was up to 1 µM, and + when the intensity was more than 1 µM but not more than 10 µM. It is to be noted that the specific affinity was evaluated to be none when the intensity was 10 µM or more. The results are shown in Table 5.

**Table 5**

| SEQ ID NO: | Affinity for phosphatidyl serine |
|---|---|
| SEQ ID NO:1 | ++ |
| SEQ ID NO:2 | ++ |
| SEQ ID NO:3 | ++ |
| SEQ ID NO:5 | ++ |
| SEQ ID NO:6 | ++ |
| SEQ ID NO:7 | + |
| SEQ ID NO:8 | + |
| SEQ ID NO:10 | + |
| SEQ ID NO:11 | + |
| SEQ ID NO:15 | ++ |
| SEQ ID NO:16 | ++ |
| SEQ ID NO:17 | ++ |
| SEQ ID NO:19 | ++ |
| SEQ ID NO:23 | ++ |

### Example 21: Evaluation of the affinity of the peptide for phosphatidyl serine (2)

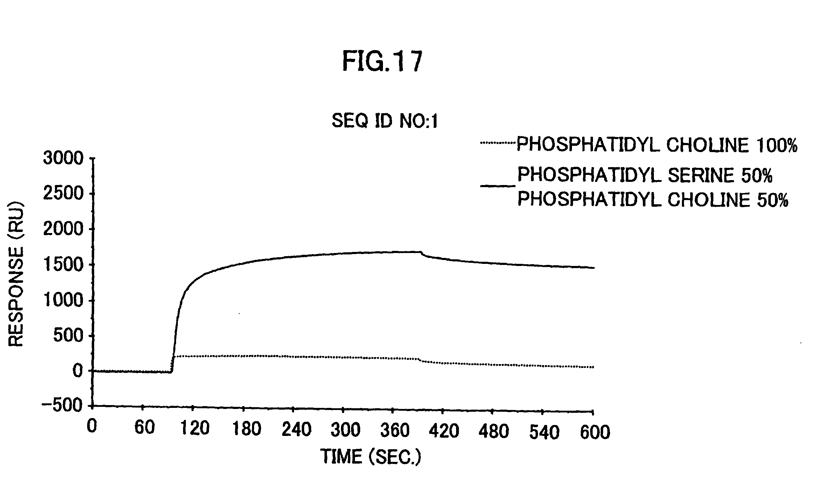

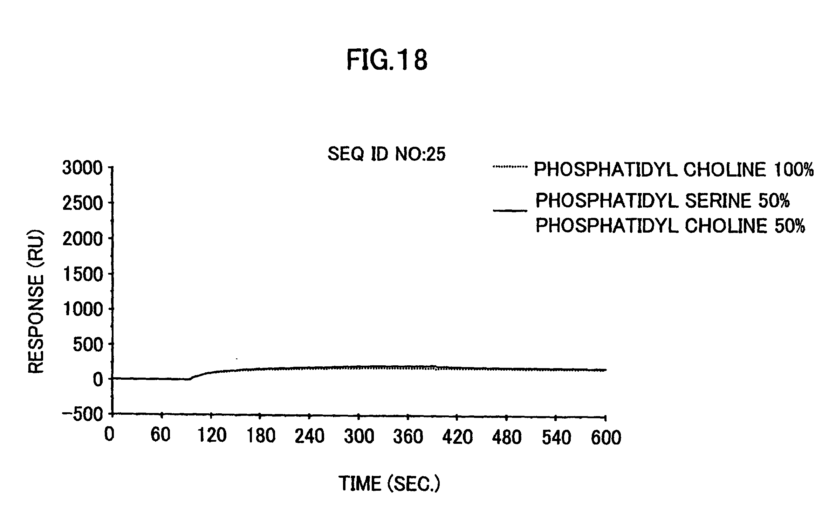

The specific affinity of the peptide for phosphatidyl serine was evaluated by means of surface plasmon resonance (SPR) using Biacore 2000 (manufactured by Biacore). To be more specific, phosphatidyl choline was immobilized on flow cell 1, and 50% of phosphatidyl serine and 50% of phosphatidyl choline were immobilized on flow cell 2 of HPA chip (manufactured by Biacore), and ability of the peptide to bind to the phospholipid was evaluated in the presence of 0.1 mg/mL BSA to thereby evaluate the affinity. It was then found that the binding of the peptide SEQ ID NO:1 to the flow cell 2 having 50% of phosphatidyl serine and 50% of phosphatidyl choline immobilized thereto was stronger than that of the flow cell 1 having phosphatidyl serine immobilized thereto, and the affinity of the peptide specific for the phosphatidyl serine was thereby indicated (FIG. 17). In contrast, the peptide of SEQ ID NO:25 wherein the sequence of SEQ ID NO:1 had been randomized showed no binding to neither the flow cell 1 having phosphatidyl choline immobilized thereto nor the flow cell 2 having 50% of phosphatidyl serine and 50% of phosphatidyl choline immobilized thereto, indicating the absence of the affinity (FIG. 18).

### Example 22: Correlation between the amount of phosphatidyl serine translocation and the introduction efficiency (selectivity)

In order to elucidate the correlation between the level of the gene-introducing ability of the peptide and the affinity for phosphatidyl serine, an investigation was conducted by using cells exhibiting different amount of phosphatidyl serine translocation to the cell surface. To be more specific, the cells used were Vero, A549, and T24 cells, and the luciferase-expressing plasmid prepared in Example 7 was introduced in the cells by using the peptide of SEQ ID NO:1 prepared in Example 1 in accordance with the description of Example 9 to thereby measure luciferase activity. In the meanwhile, the amount of phosphatidyl serine translocated to the outer surface of the cell membrane of the cells was measured by labeling the cells with FITC-labeled ANNEXIN V (ANNEXIN V-FITC, manufactured by Pharmingen) in accordance with the manual attached thereto, and conducting flow cytometry (FACS Calibur, manufactured by Becton Dickinson). To be more specific, the cells that had been scraped from the culture flask were mixed with the solution of the FITC-labeled ANNEXIN V, and the cells were measured for their FITC fluorescent intensity by flow cytometry. Average fluorescent intensity of each cell was then designated the amount of phosphatidyl serine translocation for each cell line.

Correlation was then found between the amount of phosphatidyl serine translocation (amount of ANNEXIN V binding) and the gene-introducing activity of the peptide vector as shown in Table 4. For the purpose of reference, the procedure as described above was repeated by using lipofectin (manufactured by Life Technologies Oriental) which is a commercially available plasmid-introducing agent in accordance with the attached protocol. It was then found that the plasmid was equally introduced in every type of cells, indicating the absence of the specific recognition of the phosphatidyl serine.

**Table 6**

| | | Luciferase activity (cps/mg protein) | |
|---|---|---|---|
| Cell | Amount of Annexin-V bound | Peptide (SEQ ID NO:1) | Lipofectin |
| Vero | 165 | 2.5 x 10⁶ | 1.1 x 10⁶ |
| A549 | 82 | 8.7 x 10⁴ | 8.4 x 10⁵ |
| T24 | 32 | 7.8 x 10³ | 1.1 x 10⁶ |

### Example 23: Correlation between the amount of phosphatidyl serine translocation and the introduction efficiency (2)

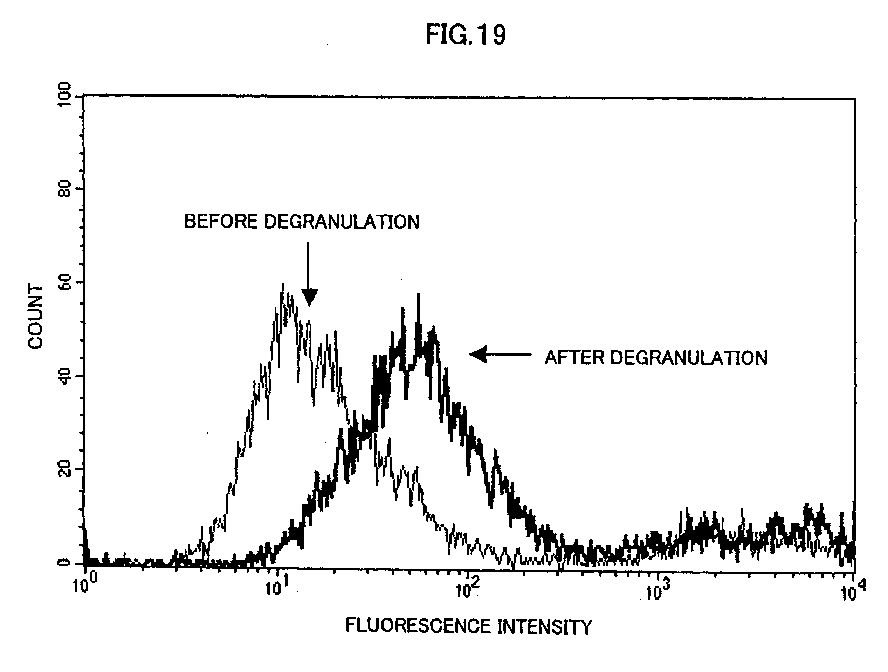

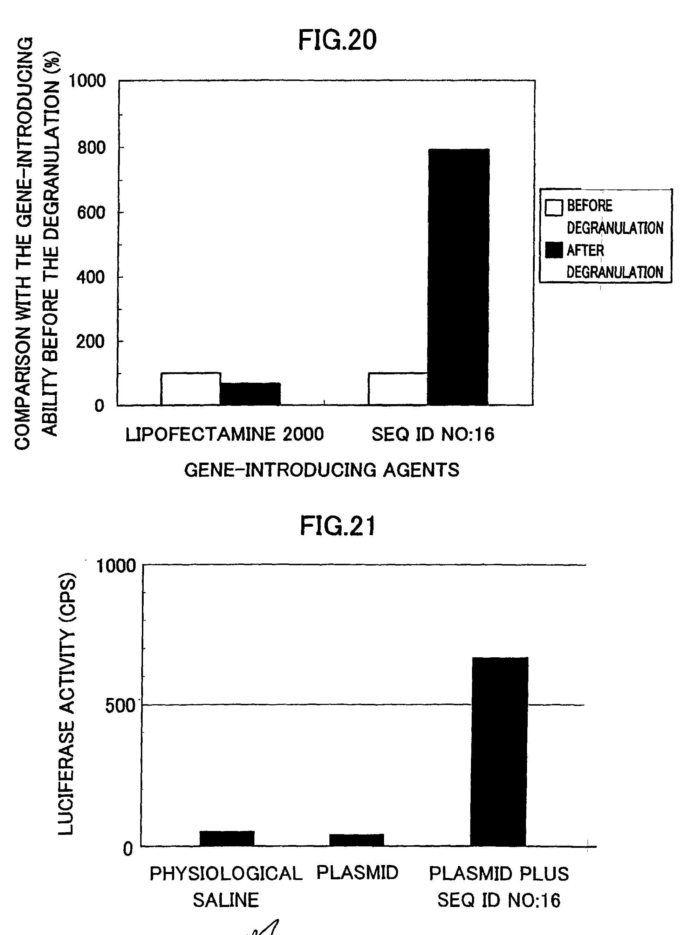

In order to elucidate the correlation between the gene-introducing ability of the peptide and the affinity for the phosphatidyl serine, nucleic acid-introducing ability of the peptide was investigated by using the cells wherein the phosphatidyl serine had not been translocated, and the cells wherein the phosphatidyl serine has been translocated by stimulating the cell. The cell used was RBL-2H3 cell from rat basophil (purchased from ATCC), and this cell was sensitized with anti-DNP mouse monoclonal IgE antibody (manufactured by SIGMA) and degranulated with DNP-BSA (manufactured by Calbiochem). Luciferase gene was introduced to such cell by using the peptide of SEQ ID NO:16 in accordance with the procedure described in Example 9 to thereby measure the luciferase activity. To be more specific, RBL-2H3 cells were inoculated in a 24 well plate at a rate of 3 x 10⁵ cells/well, and after adding anti-DNP mouse monoclonal IgE antibody to a concentration of 100 ng/mL, the cells were cultivated for 24 hours. After washing the cells twice with PBS, DNP-BSA was added to 10 ng/mL to cause degranulation for 45 minutes. After removing the culture medium and washing the well with physiological saline, opti-MEM having a concentration of the luciferase-expressing plasmid prepared in Example 7 of 1 µg/ mL and a concentration of the peptide of SEQ ID NO:16 prepared in Example 1 of 2.5 µM was added, and cultivation was continued for 5 hours. The medium was then replaced with MEM supplemented with 15% inactivated FBS (having NEAA and Na·Pyr added thereto), and incubation was continued in 5% CO₂ atmosphere at 37°C for 1 day. The cells were then scraped off, and evaluated for their luciferase activity by the procedure described in Example 9. In the meanwhile, amount of phosphatidyl serine translocated in the RBL-2H3 cell by degranulation was measured by the procedure as described below, namely, by inoculating the RBL-2H3 cell in a 6 well culture plate (manufactured by NALGEN NUNC) at a rate of 1.5 x 10⁶ cells per well, adding anti-DNP mouse monoclonal IgE antibody to a concentration of 100 ng/mL, and cultivating in MEM supplemented with 15% inactivated FBS (having NEAA and Na·Pyr added thereto) in 5% CO₂ atmosphere at 37°C for 24 hours. After washing the wells twice with PBS, DNP-BSA was added to 10 ng/mL to thereby cause degranulation for 45 minutes. After scraping off the cells, the cells were labeled with FITC-labeled ANNEXIN V (manufactured by MBL) in accordance with the procedure described in the attached manual, and the activity was measured by flow cytometry. It was then found that the cells stimulated for degranulation had the phosphatidyl serine translocated to its surface (FIG. 19), and the gene-introducing ability of the peptide into the RBL-2H3 cell that had been stimulated for degranulation was significantly high compared to the case of the undegranulated cell (FIG. 20). It is to be noted that the gene-introducing ability of LipofectAMINE 2000 which is a commercially available gene-introducing agent was equivalent or slightly lower in the case of the degranulated cell compared to the case of the cell before the degranulation.

### Example 24: Evaluation of nucleic acid-introducing ability into cell of the peptide (6)

In order to demonstrate the in vivo gene-introducing ability of the peptide, nucleic acid-introducing ability of the peptide was examined by using an ascites cancer model animal having Meth-A mouse sarcoma cells transplanted thereto. To be more specific, 4 x 10⁶ Meth-A cells were transplanted in the abdominal cavity of BALB/c mouse (purchased from Charles River JAPAN), and after 4 days, a mixture of 30 µg of the luciferase-expressing plasmid prepared in Example 7 and 113 nmol of the peptide of SEQ ID NO:16 prepared in Example 1 was administered to the abdominal cavity of the animal. The mouse was killed after another 1 day, and Meth-A cell in the abdominal cavity was recovered to thereby measure the luciferase activity. It was then found that, as shown in FIG. 21, while no expression of luciferase was found in the contrast mouse which had been administered solely with physiological saline or in the contrast mouse which had been administered only with 30 µg luciferase-expressing plasmid, luciferase was found to be expressed in the mouse which had been administered with a mixture of 30 µg of the luciferase-expressing plasmid and 113 nmol of the peptide of SEQ ID NO:16 to its abdominal cavity.

### Example 25: Evaluation of nucleic acid-introducing ability into cell of the peptide (8)

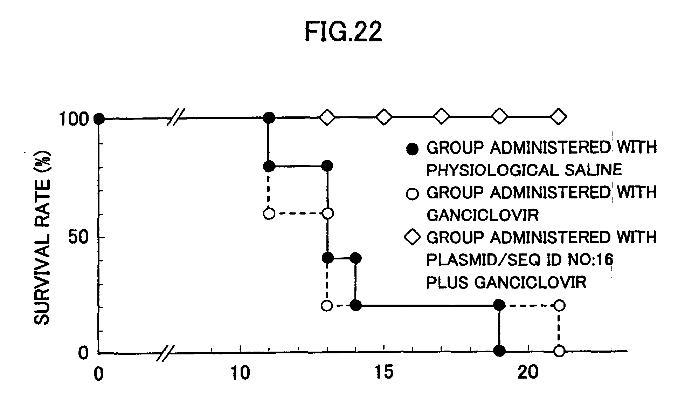

In order to demonstrate the pharmacological effects of the in vivo gene introduction by using the peptide, anti tumor action realized by the increase of GCV sensitivity by the introduction of HSV-tk gene was examined by using the model animal having Lewis lung carcinoma cell (mouse lung cancer cell) inoculated in its abdominal cavity. To be more specific, 1 x 10⁵ Lewis lung carcinoma cells were transplanted in the abdominal cavity of a C57BL/6 mouse (purchased from Charles River JAPAN), and a mixture of 10 µg of the HSV-tk-expressing plasmid prepared in Example 7 and 40 nmol of the peptide of SEQ ID NO:16 prepared in Example 1 was administered in the abdominal cavity after the cell transplantation. The mouse was also administered with GCV at a dose of 30 mg/kg/day from 1st to 8th day after the transplantation. As shown in FIG. 22, it was then found that while the average survival period of the mice was 14 days both in the case of the group administered with the physiological saline and in the case of the group administered only with GCV at a dose of 30 mg/kg/day, all mice were alive 20 days after the cell transplantation in the case of the group administered with the mixture of 10 µg of the HSV-tk-expressing plasmid and 40 nmol of the peptide of SEQ ID NO:16 followed by the administration of the GCV at a dose of 30 mg/kg/day.

### Example 26: Evaluation of toxicity of the peptide

The peptide of the present invention was administered to a mouse from its tail vein to thereby evaluate its toxicity. The mouse used was a female BALB/c mouse of 5 week old, and the peptide was used at a dose of 5 mg/kg. The peptide (SEQ ID NO:16) of the present invention was found to induce no significant effects in the mouse, demonstrating that no aggregates were formed in the blood by the peptide of the present invention, and that the peptide of the present invention is highly safe.

### Comparative Example 1

A peptide (SEQ ID NO:25) having an amino acid composition which is the same as that of the peptide of SEQ ID NO:1 but with an utterly random amino acid sequence, hence a sequence which does not include "the sequence of 18 amino acids exhibiting the four sided structure of the present invention" was evaluated for its nucleic acid-introducing ability by the procedure described in Example 9. This peptide was also evaluated for its affinity for phosphatidyl serine by the procedure described in Example 20. The results indicate that this peptide had neither the nucleic acid-introducing ability nor the affinity for phosphatidyl serine.

CD spectrum was also measured by the procedure described in Example 5. The results indicate that no α-helix structure is found even in the presence of SDS. Comparative Example 2

SEQ ID NO:26 which was prepared by conducting amino acid substitution in the peptide of SEQ ID NO:1 so that the sequence does not include "the sequence of 18 amino acids exhibiting the four sided structure of the present invention" was evaluated for the nucleic acid-introducing ability by the procedure described in Example 9. The fluorescent count was less than 10,000, and no nucleic acid-introducing ability was found.

### Comparative Example 3

Polylysine (average molecular weight: 11,000) was evaluated for its affinity for phosphatidyl serine by the procedure described in Example 20. It was then found that polylysine had no affinity for phosphatidyl serine. Polylysine was also evaluated for absorption in BSA solution by the procedure described in Example 4. The value measured was 1 or higher, and formation of aggregates was thus confirmed.

### Comparative Example 4

4₆ (Niidome, T et al., J.Biol.Chem., Vol.272, 15307 (1997)) (the peptide of SEQ ID NO:27) which is an amphipathic basic peptide having α-helix structure was evaluated for its affinity for phosphatidyl serine by the procedure described in Example 20. It was then found that this peptide had no affinity for phosphatidyl serine. This peptide was also evaluated for absorption in BSA solution by the procedure described in Example 4. The value measured was 1 or higher, and formation of aggregates was thus confirmed.

### Comparative Example 5

4₆ described in Comparative Example 4 (the peptide of SEQ ID NO:27) was administered to a mouse by the procedure described in Example 26. The mouse died immediately after the administration.

It was estimated that this result reflected the situation that this peptide is an amphipathic basic peptide having α-helix structure which easily forms aggregates in blood and which exhibits serious toxicity upon administration to an animal.

### INDUSTRIAL APPLICABILITY OF THE INVENTION

The peptide of the present invention is useful as a peptide vector since it has ability of binding to a nucleic acid and ability of introducing the nucleic acid into a cell. Since the peptide takes α-helix structure only in the presence of a particular substance, it does not substantially form aggregates in serum and remains highly soluble. In addition, when the peptide binds to a nucleic acid, the nucleic acid is stable since it is imparted with nuclease resistance. Furthermore, the peptide has affinity for phosphatidyl serine, and therefore, it can selectively introduce the nucleic acid to the cell, tissue, or organ at the site where the so called immune response has taken place, for example, by inflammation, cell activation or cytotoxicity by immunocompetent cell, or apoptosis, the site where the cells have become malignantly transformed through abnormal cell division, the site where cytotoxicity of the cells constituting blood vessel have proceeded by the progress of blood coagulation or arterial sclerosis, the site where cytotoxic reaction has proceeded by super oxide, the site where cell activation and/or cytotoxic reaction has proceeded by a protease, and therefore, the nucleic acid can be administered at a reduced dose with reduced side effects.

The vector of the present invention is a reagent containing the peptide of the present invention with a substance which binds to such peptide, and in particular a nucleic acid or other physiologically active substance, and accordingly, the vector shares the characteristic feature with the peptide of the present invention. Therefore, no substantial aggregates are formed in the serum and solubility is high. When the vector of the present invention contains a nucleic acid, the nucleic acid is imparted with nuclease resistance. The vector of the present invention also has affinity for phosphatidyl serine, and the vector is introduced in cell, tissue, or organ, and therefore, the physiologically active substance can be administered at a reduced dose with reduced side effects.

The delivery process of the present invention is a method for delivering a drug into a cell, or a drug delivery system which is based on a completely new idea. This delivery process uses a substance which has a specific affinity for phosphatidyl serine in the presence of serum albumin for the carrier to deliver the substance which binds to said substance, and in particular, a nucleic acid or other physiologically active substance, and therefore, selective incorporation of the physiologically active substance, and preferably a nucleic acid, is promoted in the cell, tissue, or organ to result in an enhanced action of the substance.

In other words, the present invention provides a novel peptide, a novel vector, and a novel composition for gene therapy which is useful in preventing or treating a disease associated with immunoresponse, cancer, arterial sclerosis, blood coagulation disorder, viral disease, and inflammation.

## Claims

1. A peptide including a sequence comprising 18 amino acids, wherein
said sequence of 18 amino acids is constituted from alternately arranged two hydrophobic sides and two hydrophilic sides in α-helix structural model depicted by Edmundson wheel plots,
one of said hydrophobic sides comprises 5 to 7 amino acids and 80 mole% or more of this side comprises hydrophobic amino acids,
one of said hydrophilic sides comprises 5 to 6 amino acids, and 80 mole% or more of this side comprises hydrophilic amino acids, and 50 mole% or more of this side comprises an amino acid selected from arginine and lysine,
the other side of said hydrophobic sides comprises 2 to 4 hydrophobic amino acids, and
the other side of said hydrophilic sides comprises 3 to 5 amino acids, and 80 mole% or more of this side comprises hydrophilic amino acids.

2. A peptide according to claim 1 wherein said peptide comprises 20 or more amino acids in total; opposite ends of the peptide constitutes N and C terminals; and any 18 consecutive amino acids in the peptide excluding the amino acids at opposite ends constitutes said sequence of 18 amino acids.

3. A peptide according to claim 1 or 2 wherein the amino acids at the N and C terminals are a hydrophilic amino acid.

4. A peptide according to any one of claims 1 to 3 wherein any sequence comprising 18 consecutive amino acids in the following amino acid sequence constitutes said sequence of 18 amino acids
X2-X3-X4-X5-X6-X7-X8-X9-X10-X11-X12-X13-X14-X15-X16- X17-X18-X19-X20-X21-X22-X23-X24-X25-X26-X27-X28-X29- X30-X31-X32-X33-X34-X35-X36,
provided that,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
X3, X10, X12, X21, X28, and X30 are independently a member selected from a hydrophobic amino acid, a neutral hydrophilic amino acid and a basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid.

5. A peptide according to any one of claims 1 to 4 wherein said peptide comprises the following amino acid sequence:
X1-X2-X3-X4-X5-X6-X7-X8-X9-X10-X11-X12-X13-X14-X15- X16-X17-X18-X19-X20-X21-X22-X23-X24-X25-X26-X27-X28- X29-X30-X31-X32-X33-X34-X35-X36-X37,
provided that
X1 and X37 are a hydrophilic amino acid,
in each of "X4, X8, X11, X15, and X19", "X8, X11, X15, X19, and X22", "X11, X15, X19, X22, and X26", "X15, X19, X22, X26, and X29", and "X19, X22, X26, X29, and X33", at least 4 amino acids in the 5 amino acids are a hydrophobic amino acid,
X3, X10, X12, X21, X28 and X30 are independently a member selected from a hydrophobic amino acid, a neutral hydrophilic amino acid, and basic hydrophilic amino acid,
in each of "X2, X5, X9, X13, and X16", "X5, X9, X13, X16, and X20", "X9, X13, X16, X20, and X23", "X13, X16, X20, X23, and X27", "X16, X20, X23, X27, and X31", and "X20, X23, X27, X31, and X34", at least 4 amino acids in the 5 amino acids are a neutral hydrophilic amino acid or a basic hydrophilic amino acid, at least 3 amino acids of which being arginine or lysine,
X6, X17, X24, and X35 are independently a hydrophobic amino acid, and
X7, X14, X18, X25, X32, and X36 are independently a neutral hydrophilic amino acid or a basic hydrophilic amino acid; and wherein,
the amino acids of from X2 to X36 may include deletion, addition, insertion, or substitution as long as at least said sequence of 18 amino acids is conserved in consecutive form.

6. A peptide according to claim 5 wherein X1 to X37 are the following amino acids:
X1 is threonine,
X37 is serine,
X2, X5, X9, X20, X23, and X27 are independently arginine or lysine,
X3 and X21 are independently a member selected from tyrosine, phenylalanine, serine and arginine,
X4, X17, X22, and X35 are independently leucine,
X6, X15, X24, and X33 are independently leucine or isoleucine,
X7, X13, X25, and X31 are independently histidine or arginine,
X8 and X26 are independently proline,
X10 and X28 are independently a member selected from serine, arginine, and leucine,
X11 and X29 are independently tryptophan or leucine,
X12 and X30 are independently a member selected from valine, leucine and serine,
X14 and X32 are independently a member selected from glutamine, asparagine and arginine,
X16 and X34 are independently alanine or arginine,
X18 is a member selected from arginine, lysine and serine,
X19 is leucine or threonine, and
X36 is arginine or serine; and wherein
the amino acids of from X2 to X36 may include deletion, addition, insertion, or substitution as long as at least said sequence of 18 amino acids is conserved in consecutive form.

7. A peptide having the amino acid sequence of any one of SEQ ID NO:1 to SEQ ID NO:24.

8. A peptide of SEQ ID NO:16 or SEQ ID NO:19.

9. A vector for gene therapy containing the peptide of any one of claims 1 to 8.

10. A composition for gene therapy containing the peptide of any one of claims 1 to 8 and a plasmid including herpes simplex virus thymidine kinase gene.

11. A composition for gene therapy according to claim 10 further comprising ganciclovir or acyclovir, said composition being used for treating or preventing cancer, tumor, arterial sclerosis, restenosis after percutaneous transluminal coronary angioplasty, or GVHD.

12. A method for treating or preventing cancer, tumor, arterial sclerosis, restenosis after percutaneous transluminal coronary angioplasty, or GVHD **characterized in that** said method comprises administering ganciclovir or acyclovir simultaneously with or after administering the composition for gene therapy of claim 10, and continuing the administration of the ganciclovir or acyclovir at least for another 5 days.

13. A method for delivering a substance which binds to the peptide of any one of claims 1 to 8 into a cell by using the peptide as the carrier.


SEQUENCE LISTING

<110> Mochida Pharmaceutical Co.,Ltd.

<120> Novel peptide

<130> MD0599

<160> 30

<210> 1

<211> 37

<212> PRT

<213> Artificial Sequence

<220>

<223> Description of Artificial Sequence:Peptide

<400> 1

Thr Arg Tyr Leu Arg Ile His Pro Arg Ser Trp Val His Gln Ile Ala Leu Arg

1               5                   10                  15

Leu Arg Tyr Leu Arg Ile His Pro Arg Ser Trp Val His Gln Ile Ala Leu Arg

    20                  25                  30                  35

Ser

<210> 2

<211> 37

<212> PRT

<213> Artificial Sequence

<220> Description of Artificial Sequence:Peptide

<400> 2

Thr Arg Tyr Leu Arg Ile His Pro Arg Ser Trp Val Arg Gln Ile Ala Leu Arg

1               5                   10                  15

Leu Arg Tyr Leu Arg Ile His Pro Arg Ser Trp Val Arg Gln Ile Ala Leu Arg

    20                  25                  30                  35

Ser

<210> 3

<211> 37

<212> PRT

<213> Artificial Sequence

<220>

<223> Description of Artificial Sequence:Peptide

<400> 3

Thr Arg Tyr Leu Arg Ile His Pro Arg Ser Trp Val His Gln Ile Arg Leu Arg

1               5                   10                  15

Leu Arg Tyr Leu Arg Ile His Pro Arg Ser Trp Val His Gln Ile Arg Leu Arg

    20                  25                  30                  35

Ser

<210> 4

<211> 37

<212> PRT

<213> Artificial Sequence

<220>

<223> Description of Artificial Sequence:Peptide

<400> 4

Thr Arg Tyr Leu Arg Ile His Pro Arg Ser Trp Leu His Gln Ile Ala Leu Arg

1               5                   10                  15

Leu Arg Tyr Leu Arg Ile His Pro Arg Ser Trp Leu His Gln Ile Ala Leu Arg

    20                  25                  30                  35

Ser

<210> 5

<211> 37

<212> PRT

<213> Artificial Sequence

<220>

<223> Description of Artificial Sequence:Peptide

<400> 5

Thr Arg Phe Leu Arg Ile His Pro Arg Ser Trp Val His Gln Ile Ala Leu Arg

1                   5                   10                  15

Leu Arg Phe Leu Arg Ile His Pro Arg Ser Trp Val His Gln Ile Ala Leu Arg

    20                  25                  30                  35

Ser

<210> 6

<211> 37

<212> PRT

<213> Artificial Sequence

<220>

<223> Description of Artificial Sequence:Peptide

<400> 6

Thr Arg Tyr Leu Arg Ile His Pro Arg Ser Trp Val His Asn Ile Ala Leu Arg

1                   5                   10                  15

Leu Arg Tyr Leu Arg Ile His Pro Arg Ser Trp Val His Asn Ile Ala Leu Arg

    20                  25                  30                  35

Ser

<210> 7

<211> 37

<212> PRT

<213> Artificial Sequence

<220>

<223> Description of Artificial Sequence:Peptide

<400> 7

Thr Arg Tyr Leu Arg Ile His Pro Arg Ser Trp Val His Gln Ile Ala Leu Lys

1               5                   10                  15

Leu Lys Tyr Leu Arg Ile His Pro Arg Ser Trp Val His Gln Ile Ala Leu Arg

    20                  25                  30                  35

Ser

<210> 8

<211> 37

<212> PRT

<213> Artificial Sequence

<220>

<223> Description of Artificial Sequence:Peptide

<400> 8

Thr Arg Tyr Leu Arg Ile His Pro Lys Ser Trp Val His Gln Ile Ala Leu Arg

1               5                   10                  15

Leu Arg Tyr Leu Lys Ile His Pro Arg Ser Trp Val His Gln Ile Ala Leu Arg

    20                  25                  30                  35

Ser

<210> 9

<211> 37

<212> PRT

<213> Artificial Sequence

<220>

<223> Description of Artificial Sequence:Peptide

<400> 9

Thr Arg Tyr Leu Arg Ile His Pro Arg Ser Trp Val His Gln Ile Ala Leu Arg

1               5                   10                  15

Thr Arg Tyr Leu Arg Ile His Pro Arg Ser Trp Val His Gln Ile Ala Leu Arg

    20                  25                  30                  35

Ser

<210> 10

<211> 37

<212> PRT

<213> Artificial Sequence

<220>

<223> Description of Artificial Sequence:Peptide

<400> 10

Thr Arg Ser Leu Arg Ile His Pro Arg Ser Trp Val His Gln Ile Ala Leu Arg

1               5                   10                  15

Leu Arg Ser Leu Arg Ile His Pro Arg Ser Trp Val His Gln Ile Ala Leu Arg

    20                  25                  30                  35

Ser

<210> 11

<211> 37

<212> PRT

<213> Artificial Sequence

<220>

<223> Description of Artificial Sequence:Peptide

<400> 11

Thr Arg Tyr Leu Arg Ile His Pro Arg Leu Trp Val His Gln Ile Ala Leu Arg

1               5                   10                  15

Leu Arg Tyr Leu Arg Ile His Pro Arg Leu Trp Val His Gln Ile Ala Leu Arg

    20                  25                  30                  35

Ser

<210> 12

<211> 37

<212> PRT

<213> Artificial Sequence

<220>

<223> Description of Artificial Sequence:Peptide

<400> 12

Thr Arg Tyr Leu Arg Ile His Pro Arg Ser Trp Val His Gln Ile Ala Leu Ser

1               5                   10                  15

Leu Arg Tyr Leu Arg Ile His Pro Arg Ser Trp Val His Gln Ile Ala Leu Ser

    20                  25                  30                  35

Ser

<210> 13

<211> 37

<212> PRT

<213> Artificial Sequence

<220>

<223> Description of Artificial Sequence:Peptide

<400> 13

Thr Arg Tyr Leu Arg Ile His Pro Arg Ser Leu Val His Gln Ile Ala Leu Arg

1               5                   10                  15

Leu Arg Tyr Leu Arg Ile His Pro Arg Ser Leu Val His Gln Ile Ala Leu Arg

    20                  25                  30                  35

Ser

<210> 14

<211> 37

<212> PRT

<213> Artificial Sequence

<220>

<223> Description of Artificial Sequence:Peptide

<400> 14

Thr Arg Tyr Leu Arg Ile His Pro Arg Ser Trp Ser His Gln Ile Ala Leu Arg

1               5                   10                  15

Leu Arg Tyr Leu Arg Ile His Pro Arg Ser Trp Ser His Gln Ile Ala Leu Arg

    20                  25                  30                  35

Ser

<210> 15

<211> 37

<212> PRT

<213> Artificial Sequence

<220>

<223> Description of Artificial Sequence:Peptide

<400> 15

Thr Arg Tyr Leu Arg Ile Arg Pro Arg Ser Trp Val Arg Gln Ile Ala Leu Arg

1               5                   10                  15

Leu Arg Tyr Leu Arg Ile Arg Pro Arg Ser Trp Val Arg Gln Ile Ala Leu Arg

    20                  25                  30                  35

Ser

<210> 16

<211> 37

<212> PRT

<213> Artificial Sequence

<220>

<223> Description of Artificial Sequence:Peptide

<400> 16

Thr Arg Tyr Leu Arg Leu His Pro Arg Ser Trp Val His Gln Leu Ala Leu Arg

1               5                   10                  15

Leu Arg Tyr Leu Arg Leu His Pro Arg Ser Trp Val His Gln Leu Ala Leu Arg

    20                  25                  30                  35

Ser

<210> 17

<211> 37

<212> PRT

<213> Artificial Sequence

<220>

<223> Description of Artificial Sequence:Peptide

<400> 17

Thr Arg Ser Leu Arg Ile His Pro Arg Leu Trp Val His Gln Ile Ala Leu Arg

1               5                   10                  15

Leu Arg Ser Leu Arg Ile His Pro Arg Leu Trp Val His Gln Ile Ala Leu Arg

    20                  25                  30                  35

Ser

<210> 18

<211> 37

<212> PRT

<213> Artificial Sequence

<220>

<223> Description of Artificial Sequence:Peptide

<400> 18

Thr Arg Tyr Leu Arg Ile His Pro Arg Ser Trp Leu His Gln Ile Ala Leu Arg

1               5                   10                  15

Thr Arg Tyr Leu Arg Ile His Pro Arg Ser Trp Leu His Gln Ile Ala Leu Arg

    20                  25                  30                  35

Ser

<210> 19

<211> 37

<212> PRT

<213> Artificial Sequence

<220>

<223> Description of Artificial Sequence:Peptide

<400> 19

Thr Arg Tyr Leu Arg Leu His Pro Arg Ser Trp Leu His Gln Leu Ala Leu Arg

1               5                   10                  15

Leu Arg Tyr Leu Arg Leu His Pro Arg Ser Trp Leu His Gln Leu Ala Leu Arg

    20                  25                  30                  35

Ser

<210> 20

<211> 37

<212> PRT

<213> Artificial Sequence

<220>

<223> Description of Artificial Sequence:Peptide

<400> 20

Thr Arg Tyr Leu Arg Leu His Pro Arg Ser Trp Val His Gln Leu Ala Leu Arg

1               5                   10                  15

Thr Arg Tyr Leu Arg Leu His Pro Arg Ser Trp Val His Gln Leu Ala Leu Arg

    20                  25                  30                  35

Ser

<210> 21

<211> 37

<212> PRT

<213> Artificial Sequence

<220>

<223> Description of Artificial Sequence:Peptide

<400> 21

Thr Lys Tyr Leu Lys Ile His Pro Lys Ser Trp Val His Gln Ile Ala Leu Arg

1               5                   10                  15

Leu Lys Tyr Leu Lys Ile His Pro Lys Ser Trp Val His Gln Ile Ala Leu Arg

    20                  25                  30                  35

Ser

<210> 22

<211> 37

<212> PRT

<213> Artificial Sequence

<220>

<223> Description of Artificial Sequence:Peptide

<400> 22

Thr Arg Arg Leu Arg Ile His Pro Arg Arg Trp Val His Arg Ile Ala Leu Arg
1               5                   10                  15
Leu Arg Arg Leu Arg Ile His Pro Arg Arg Trp Val His Arg Ile Ala Leu Arg
    20                  25                  30                  35
Ser

<210> 23
<211> 31
<212> PRT
<213> Artificial Sequence
<220>
<223> Description of Artificial Sequence:Peptide
<400> 23
Thr Ile His Pro Arg Ser Trp Val His Gln Ile Ala Leu Arg Leu
1               5                   10                  15
Arg Tyr Leu Arg Ile His Pro Arg Ser Trp Val His Gln Ile Ala Ser
                20                  25                  30

<210> 24
<211> 20
<212> PRT
<213> Artificial Sequence

<220>

<223> Description of Artificial Sequence:Peptide

<400> 24

Thr Leu Arg Tyr Leu Arg Ile His Pro Arg Ser Trp Leu His Gln

1               5                   10                  15

Ile Ala Leu Arg Ser

                20

<210> 25

<211> 37

<212> PRT

<213> Artificial Sequence

<220>

<223> Description of Artificial Sequence:Peptide

<400> 25

Thr Ile Arg Tyr Arg Pro Ser His Gln Ile Arg Leu Arg Ala Val Leu His Leu

1               5                   10                  15

Trp Ile Arg Tyr Arg Pro Ser His Gln Ile Arg Leu Arg Ala Val Leu His Trp

    20                  25                  30                  35

Ser

<210> 26

<211> 37

<212> PRT

<213> Artificial Sequence

<220>

<223> Description of Artificial Sequence:Peptide

<400> 26

Thr Arg Tyr Leu Arg Ile His Pro Arg Ser Trp Val Leu Gln Ile Ala Leu Arg

1               5                   10                  15

Leu Arg Tyr Leu Arg Ile His Pro Arg Ser Trp Val Leu Gln Ile Ala Leu Arg

    20                  25                  30                  35

Ser

<210> 27

<211> 24

<212> PRT

<213> Artificial Sequence

<220>

<223> Description of Artificial Sequence:Peptide

<400> 27

Leu Ala Arg Leu Leu Ala Arg Leu Leu Ala Arg Leu Leu Arg Ala Leu Leu Arg

1               5                   10                  15

Ala Leu Leu Arg Ala Leu

    20

<210> 28

<211> 111

<212> DNA

<213> Artificial Sequence

<220>

<223> Description of Artificial Sequence:DNA

<400> 28

actcgttatc ttcgcattca tcctcgaagt tgggttcacc aaatagctct gagactacgg 60

tacttacgaa ttcacccacg tagctgggtt caccaaatag ctctgcgttc t 111

<210> 29

<211> 111

<212> DNA

<213> Artificial Sequence

<220>

<223> Description of Artificial Sequence:DNA

<400> 29

actcgttatc ttcgccttca tcctcgaagt tgggttcacc aactagctct gagactacgg 60

tacttacgac ttcacccacg tagctgggtt caccaactag ctctgcgttc t 111

<210> 30

<211> 111

<212> DNA

<213> Artificial Sequence

<220>

<223> Description of Artificial Sequence:DNA

<400> 30

actcgttatc ttcgccttca tcctcgaagt tggcttcacc aactagctct gagactacgg 60

tacttacgac ttcacccacg tagctggctt caccaactag ctctgcgttc t 111